# EUROPEAN PATENT APPLICATION

(11) **EP 4 843 556 A1**
(43) Date of publication of application: **25.11.2026**
(21) Application number: 25178471.6
(22) Date of filing: 23.05.2025
(51) Int. Cl.: A61K 8/44, A61Q 5/08, A61Q 5/10

(54) **BLEACH COMPOSITION COMPRISING A MIXTURE OF THREE CHELATANTS**

(71) Applicant: Wella Germany GmbH, 64295 Darmstadt (DE)
(72) Inventor: Alam, Ehsanul, 64295 Darmstadt (DE); Pryadilova, Olga, 64295 Darmstadt (DE); Richter, Marcus, 64295 Darmstadt (DE); Schmitt, Manfred, 64295 Darmstadt (DE)
(74) Representative: Dierkes, Thorsten

(57) **Abstract**

The present invention is directed to embodiments of a bleach composition comprising at least an oxidizer, an alkalizer ant a mixture of three chelants, EDDS, MGDA and EDTA. The bleach composition delivers improved beaching results to keratin fiber including high lift, low keratin fiber damage and a lowered temperature of oxidation.

## Description

### Technical Field

The present invention is directed to bleach composition embodiments of a mixture of three chelants, EDDS, MGDA and EDTA that deliver improved beaching results to keratin fiber including high lift, low keratin fiber damage and a lowered temperature of oxidation. These results provide advantageous aspects to the consumer.

### Background

Coordination of hair color with fashion trends as well as with personal preference represents an important feature of modern cosmetics. Whether the desired hair color of the moment is to be platinum, Nordic blond, strawberry roan, fiery red, auburn, chestnut, black or fanciful such as green or pink, modern cosmetics can achieve it. This requires elimination of the current color whether it is from color bodies such as natural melanin arising from a mixture of eumelanin and phenomelanin or from artificial substances such as precursor-coupler dyes or direct dyes. The elimination calls for bleaching these color bodies to colorless entities and the change is completed by introduction of the new color or colors onto or into the hair.

There is a cost, however. Bleaching or oxidation of the color bodies carries with it oxidation of the keratin protein of the hair. Although the "lift" of color provided by the color body bleaching achieves a blank canvas on which to "paint" the new color, the attendant keratin oxidation leads to problems, not the least of which is scission of the disulfide and amide bonds of the keratin protein. The visible results can be fly-away hair, facile hair strand breakage, rough feeling hair, and hair follicle damage. Moreover, the bleaching/oxidation process can harm the skin of the scalp through direct oxidation and/or by the heat generated by the oxidation process.

The usual oxidant for the procedure involving color change is hydrogen peroxide. While it is stable at acidic pH, it does not readily function as an oxidizer at this pH. Increasing the pH to basic enables the facile oxidation function of hydrogen peroxide but it readily decomposes at basic pH. Moreover, an oxidation medium at a pH of about 11, is deleterious to the keratin protein and skin of the scalp and causes the problems discussed above.

Attempts to ameliorate these untoward results of hair color change are plentiful. Included for example are use of oil and petroleum jelly to protect keratin fibers and skin and use of an oxidizing complex such as carbonate and/or bicarbonate combined with hydrogen peroxide to produce percarbonate/perbicarbonate oxidizing moieties that are short-lived and can effectively function at lower pH such as 8 to 9. These kinds of techniques are typically associated with oxidative precursor-coupler dye procedures which usually follow the bleaching of the hair to produce the colorless entities from the pre-existing natural or artificial color bodies of the hair.

The bleaching technique attendant to the oxidative dye procedure and as an independent procedure done to "lift" the pre-existing color also has these untoward results. "Persalts" such as persulfate, percarbonate, perborate, hydrochlorite, and the like manage the oxidation and to some extent avoid the fast oxidative reaction typically associated with hydrogen peroxide.

Nevertheless, all of these oxidation techniques require a basic medium. Generation of this high pH is typically accomplished by use of an alkaline material such as ammonia, ammonium hydroxide, organic amine, silicate, metasilicate and similar materials. These alkaline materials are also deleterious to keratin protein and scalp skin over and above the difficulties caused by the oxidation moiety at basic pH.

As is expected, any of these combinations of basic pH, an alkalizing agent and bleach generates heat of reaction. To avoid scalding the skin of the scalp and producing runaway oxidation proceeding at high temperatures, the usual procedure for bleaching or oxidative dyeing calls for separating the head of hair into multiple sections, isolating the sections with clips and bleaching a section at a time. The bleach composition is applied to isolated horizontal swatches of a section until all horizontal swatches of a section are covered with bleach. The usual procedure involves application of the bleach from about one inch above the roots to the tip ends, complete this procedure for the entire section and then with a lower concentration of bleach, cover the roots. Use of the lower concentration on the roots portions of the swatches compensates for the higher oxidation temperature maintained at the roots compared with the oxidation temperature at the hair ends.

Whether the bleaching is done professionally or by a consumer, the process is long, tedious and always has the above-described problems associated with the oxidative treatment of hair. In particular, weaking of the keratin fibers by oxidation of keratin disulfide and amide bonds, uncontrolled oxidation temperature and uneven bleaching are typical results no matter who does the bleaching. Uncontrolled oxidation temperature is especially troublesome for bleaching techniques because the technique does not protect the scalp or sections of hair from rapid, high temperature oxidative reactions. Such reactions have a tendency to cause scalp scalding, keratin protein scission and follicle injury.

Therefore, there is a need to improve lift and lessen keratin fiber and protein damage while at the same time managing bleaching conditions at temperatures that promote oxidation but do not rise to degrees that can harm keratin fibers and the scalp.

### Summary of the Invention

These needs are met by the present invention which is directed to a bleach composition, to a method for lightening and/or de-colorizing hair with the bleach composition and to a method for color lifting with the bleach composition in combination with a subsequent oxidative dyeing process.

The bleach composition comprises:
a) an oxidizing agent, preferably a peroxo oxidizing agent;
b) a chelate mixture consisting of ethylenediamine-N,N'disuccinic acid (EDDS), methylglycine-N,N-diacetic acid (MGDA) and ethylene diamine tetraacetic acid (EDTA wherein the weight parts ranges for EDDS, MGDA and EDTA in the chelate mixture relative to the total weight of the chelate mixture are:
   i) EDDS at a weight parts range of about 4 to about 1; and
   ii) MGDA at a weight parts range of about 3 to about 0.5; and
   iii) EDTA at a weight parts range of about 2.0 to about 0.5 ; and
c) an alkalizing agent; preferably an inorganic alkalizing agent.

The bleach composition optionally may further comprise one or more additives.

The bleach composition has a physical form of a solid or paste, preferably a solid of a particulate or powder form.

The constituent concentrations of the bleach composition comprise:
d) the oxidizing agent at about 20 to about 90 weight percent,
e) the chelate mixture at about 4 to about 16 weight percent, and
f) the alkalizing agent at about 10 to about 60 weight percent,
g) with the weight percentages of each of d, e and f being relative to the total weight of the bleach composition wherein:
   i) the bleach composition comprises the oxidizing agent, the chelate mixture and the alkalizing agent alone; or
   ii) the bleach composition comprises the oxidizing agent, the chelate mixture, the alkalizing agent and one or more additives; and the total weight percentage of the one or more additive(s) is the difference between 100 weight percent and the sum of the weight percentages of the oxidizing agent, the chelate mixture and the alkalizing agent.

The method for lightening and/or decolorizing hair comprises use of the bleach composition alone by combining it with an aqueous medium or a developer to produce an aqueous or developer semi-fluid material. The aqueous or developer semi-fluid material is applied to the hair and allowed to remain on the hair for a period of time to accomplish the desired degree of lightening and/or decolorizing and then the hair is rinsed to remove residual aqueous or developer semi-fluid material. The developer to be combined with the bleach composition is preferably aqueous hydrogen peroxide.

The method for bleaching and oxidatively dyeing the hair comprises two steps. The first step comprises applying the foregoing method for lightening and/or decolorizing hair. The second step comprises applying an oxidative dye composition of at least a precursor, a coupler and an oxidizing agent to the lightened and/or decolorized hair.

The bleach composition according to the invention delivers an improved lift and improved protection against keratin protein damage relative to bleach compositions containing one or two of the chelators of the chelate mixture. The bleach composition also delivers the highest oxidation reaction temperature of about 50° C or less.

### Brief description of the Figures

Figure 1 presents Graph 7/4 depicting the damage to lift ratio curve for EDDS.
Figure 2 presents Graph 8/5 depicting the damage to lift ratio curve for MGDA.
Figure 3 presents Graph 9/6 depicting the damage to lift ratio curve for the combination of EDDS and MGDA.
Figure 4 presents Hypothetical Graph 10 depicting the calculated damage to lift ratio curve for the combination of the individual EDDS and MGDA lift and FTIR results.
Figure 5 presents Graph 11 depicting the temperature increase of hair swatches treated with Bleach compositions.

### Definitions

Unless defined otherwise, all technical and scientific terms used herein have the same meaning as commonly understood by a person of ordinary skill in the art.

As used in the specification and the appended statements and claims, the singular forms "a," "an" and "the" include plural referents unless the context clearly dictates otherwise. Also, use of a plural term describing a thing or element includes the singular unless the context clearly dictates otherwise. For example, the term embodiments of a bleach composition includes a single embodiment of a bleach composition as well as multiple embodiments of a bleach composition.

The term "may" in the context of this application means "is permitted to" or "is able to" and is a synonym for the terms "can" and "is." The term "may" as used herein does not mean possibility or chance.

The term and/or in the context of this application means one or the other or both. For example, an aqueous solution of A and/or B means an aqueous solution of A alone, an aqueous solution of B alone and an aqueous solution of a combination of A and B. When more than two items are referred, the term and/or also means any combination of these multiple items as well as all and each.
The terms "about" and "approximately" as used herein, when referring to a numerical value or range, allow for a degree of variability in the value or range, and are understood to mean ±10%, of a stated value or of a stated limit of a range.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4. Similarly, values expressed in a range format should be interpreted in a flexible manner to include not only the numerical values explicitly recited as the limits of the range, but also to include all the individual numerical values or sub-ranges encompassed within that range as if each numerical value and sub-range were explicitly recited. For example, a range of "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range.

The terms "comprising", "comprises" and "comprised of" as used herein are synonymous with "including", "includes" or "containing", "contains", or "provides" or "providing" and are inclusive, open-ended, expansive and do not exclude additional, non-recited members, elements or method steps.

As used herein, the term "optionally" means that the corresponding substituent or thing may or may not be present. It includes both possibilities.

For the purposes of the presently claimed invention, "% by weight" or "wt.%" or "wt%" or weight percentage as used in the presently claimed invention is with respect to the total weight of the recited composition. Further, the sum of wt.% of all the compounds (components), as described herein, in the respective compositions may add up to 100 wt.% when optional non-delineated components such as water, non-reactive organic solvent and additives are included. Accordingly, all percents of ingredients or components are given as weight-percentages relative to the total weight of the composition in which the ingredients and/or components are present, unless otherwise stated.

All average molecular weights of polymers are weight-average molecular weights, unless otherwise specified.

The term "substantially free" as the term is used herein means completely or almost completely; for example, a composition that is "substantially free" of a component either has none of the component or contains such a small amount that any relevant functional property of the composition is unaffected by the presence of the small amount of the component in question. A compound that is "substantially pure" has only negligible traces of impurities present.

The term "substantial" means a significant amount such as more than a majority amount. For example, a mixture of compounds A and B in which A is present in a substantial amount means that A is present at a weight percent or number of moles that is greater than the weight percent or number of moles of B. This term also means more than a minimal characteristic, examples of which include substantial flow or substantial transformation.

The terms "essential" and "essentially" are encompassed by the terms substantial and substantially so as to cover a smaller range than the terms substantial and substantially. The terms essential and essentially relating to purity or result equal to or more than 98 % of the theoretic 100% purity or result.

The terms "Chelate", "Chelator", "Chelant", Chelating Agent", "Sequestering Agent" and "Sequestrant" are interchangeable herein and mean an organic compound that exhibits complexation with an inorganic metal ion such as copper, iron, calcium, magnesium; additionally inorganic element ions and atoms such as mercury, arsenic, lead, manganese, gold, silver, zirconium, gallium, yttrium and others may be complexed by such chelating agents although such elements are not typically found in connection with use of hair bleach.

The term gel means a liquid within a three-dimensional network that forms at least a semi-solid-like consistency in the static, quiescent state. The gel has sufficient rheologic control and/or density and/or viscosity at rest (static state) to maintain continuous integrity of the liquid gel as a coating or layer on a flat or curved surface. The gel character of the liquid means that the liquid will not spontaneously flow off a surface on which it has been coated but can be readily removed by mechanical force such as by wiping with a cloth or tissue.

The terms flowable and semi-flowable respectively mean: a) a substance that will flow like a liquid such as water or milk (e.g. flowable) and b) a substance that will flow like an aqueous latex paint or molasses at ambient temperature (e.g., semi-flowable).

Together, the terms gel and flowable mean that the substance having these characteristics is thixotropic.

The term paste means a thick, soft, viscous substance that is capable of being spread substantially contiguously upon a substrate by application of force but does not have a thixotropic, flowable or semi-flowable property and is not capable of self-spreading by gravitational force.

The term semi-fluid material means a semi-flowable to paste-like material that includes water as at least a part of the medium of the semi-fluid material.

The terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

The term "keratin fibers" means natural individual hair strands containing keratin protein. The keratin fibers or hair strands originate from such sources as scalp hair, eye-brow hair and eyelash hairs. A group of natural keratin fibers or hair strands constitutes anagenic hair such as the group of hair strands or keratin fibers on the head of a living person. Hair includes hair harvested from mammals and/or attached to mammals including human, primate, ruminant, camelid, equine, rodent and neovison including but not limited to cow, sheep, deer, goat, buffalo, lama, alpaca, camel, guanaco, vicuna, horse, antelope, moose, elk, rat, mouse, beaver, rabbit, mink, monkey, ape and similar species. Natural keratin material may include hair and fur. Keratin fibers include scalp hair, eyebrow hair and eyelash hair.

Keratin fibers may also be constructed of artificial keratin material such as non-natural and/or synthetic keratin or keratin-like protein that is configured into individual strands. The artificial keratin material may or may not contain color bodies that can be lifted to lighter colors by post exposure to an oxidizing agent. For the purposes of the present invention, the term "keratin fibers" primarily focuses on natural keratin fibers.

As used herein "anagenic hair" means hair strands that are in direct connection with a hair follicle which is in either the anagen or telogen state. Anagenic hair is present in one of these states on the scalp of a person, a human. The follicle of anagenic hair produces long chain fatty acids, so-called F-layer, which form a water resistant coating on the cuticle of the hair shaft. Joining the hair follicle channel is a sebaceous gland that secretes sebum onto the hair shaft and onto the scalp. As a strand of hair grows from the follicle and extends from the scalp, sebum produced at the follicle spreads out from the follicle and continues to coat the strand. Sebum is removed at least in part from strand ends by shampooing but is replenished by this continued production. Hair cut from a living person is no longer anagenic hair.

By "lift" (or "lift power") it is meant the amount of lightening of the natural hair pigment melanin caused by the bleach by using the bleach composition of the presently claimed invention. The amount of lift provided by different hair colouring compositions can be compared by using a human natural dark hair sample (e.g., dark hair of an individual of Chinese descent) and measuring the change of colour achieved following application of the compositions. The change in colour can be measured using well known parameters such as L*a*b* values of the CIE color spectrum. A composition can be said to provide a higher lift than another composition, when the resulting L* value or the so-called dL* value (given by L*_{final} - L*ᵢₙᵢₜᵢₐₗ) measured for a given treated sample of dark hair is higher for that composition than for the other composition, using the same experimental conditions. The denomination Level 2 (herein used interchangeably with "demi-permanent" or "tone-on-tone") and Level 3 (herein used interchangeably with "permanent") are commonly used in the hair colour trade to differentiate compositions with medium and high lift.

The term "user" means the person preparing and applying a bleach composition of the present invention. The user may be, for example, a professional hair care stylist who is different from the person on whose hair the bleach composition is to be applied. The user, for example, may also be the same as the person receiving the bleach composition, i.e., the user may be the consumer.

The following groups of terms are used throughout this application: 1) preferred, preferably and preferable; 2) more preferred, more preferably and more preferable, 3) especially more preferred, especially more preferably and especially more preferable; 4) most preferred, most preferably and most preferably; 5) especially most preferred, especially most preferably and especially most preferable; and 6) very especially most preferred, very especially most preferably and very especially most preferable. These groups convey a meaning of preference for a group of substituents, structures, moieties, components and compounds. The degree of preference is self-explanatory by the terms themselves. Within each group, the meanings of the synonyms, preferred, preferably and preferable are the same. There is no difference in meaning in the context of this application when a group is described in a particular sentence as preferred and then in another sentence this same group is described as preferably. Not all six categories of preference are used in this application to describe each and every substituent, formula, subgenus integer symbol and atom designator. In some instances, two or three categories are used while in other categories five or six categories are used. The degree of preference as expressed by these terms for members of series which progress from many to a few individually named components is self-explanatory and internally consistent for the particular series being described.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous. A particular feature, structure, or characteristic described in connection with an embodiment may not be the same as another feature, structure or characteristic of another embodiment of the presently claimed invention. Thus, appearances of the phrases "in one embodiment" or "an embodiment" or "in a preferred embodiment" in various places throughout this specification are not necessarily all referring to the same embodiment but may refer to different embodiments of the presently claimed invention. Furthermore, reference throughout this specification to "embodiment", "one embodiment" or "preferred embodiment" or "more preferred embodiment" or "most preferred embodiment" means a feature of any one or more elements and can be used in connect with different elements of the invention. Furthermore, the features, structures, or characteristics in one or more embodiments may be combined in any suitable manner, as would be apparent to a person skilled in the art from this disclosure. Furthermore, while some embodiments described herein include some, but not other features included in other embodiments, combinations of features of different embodiments are meant to be within the scope of the subject matter, and form different embodiments, as would be understood by those skilled in the art. For example, in the appended claims, any of the claimed embodiments can be used in any combination.

### Detailed Description

The present invention is directed to embodiments of a bleach composition that display improved lift, better protection against iron, copper and calcium ion interference, better protection against keratin damage, increased foaming protection and a lower peak oxidation temperature than bleach compositions of the past display. Embodiments of the bleach composition may be employed to lighten hair color, decolorize hair and as a pre-treatment step for oxidative dyeing of hair.

Embodiments of the bleach composition according to the invention comprise at least an oxidizing agent, a chelate mixture and an alkalizing agent. Embodiments of the bleach composition deliver treatment benefits including an excellent lift, protection against iron, copper and calcium interference, good keratin protection and management of a maximum temperature of oxidation reaction.

In particular, the bleach composition comprises at least:
a) an oxidizing agent;
b) a chelate mixture consisting of ethylenediamine-N,N'disuccinic acid (EDDS), methylglycine-N,N-diacetic acid (MGDA) and ethylene diamine tetraacetic acid (EDTA); and
c) an alkalizing agent.

The treatment benefits delivered by embodiments of the bleach composition according to the invention are obtained at least in part by the chelate mixture having a defined weight parts ratio range. The weight part ratio range is established by individual weight part number ranges for each of the individual chelators of the EDDS, MGDA, EDTA mixture. The individual weight part number ranges include:
a) an individual weight parts number range for EDDS which is a maximum of about 4, a minimum of about 1 and can be any fraction between 4 and 1,
b) an individual weight parts number range for MGDA which is a maximum of about 3, a minimum of about 0.5 and can be any fraction between 3 and 0.5, and
c) an individual weight parts range for EDTA which is a maximum of about 2, a minimum of about 0.5 and can be any fraction between 2 and 0.5.
These individual weight part number ranges are relative to the total weight of the mixture of chelators not the total weight of the bleach composition. The total weight of the mixture is relative to the total weight of the bleach composition as described below.

The chelate mixture weight part ratio range comprises any three part combination of the individual weight part ranges for each of EDDS, MGDA and EDTA. These individual weight part ranges include:
a) EDDS weight part range as fractions between about 4 and about 1 including 4.0, 3.9, 3.8, 3.7, 3.6, 3.5, 3.4, 3.3, 3.2, 3.1, 3.0, 2.9. 2.8. 2.7, 2.6, 2.5, 2.4, 2.3, 2.2, 2.1, 2.0, 1.9, 1.8, 1.7, 1.6, 1.5, 1.4, 1.3, 1.1 or 1.0 or any fractional variation thereof;
b) MGDA weight part range as fractions between about 3 and about 0.5 including 3.0, 2.9, 2.8, 2.7, 2.6, 2.5, 2.4, 2.3, 2.2, 2.1, 2.0, 1.9. 1.8. 1.7, 1.6, 1.5, 1.4, 1.3, 1.2, 1.1, 1.0, 0.9, 0.8, 0.7, 0.6 or 0.5 or any fractional variation thereof;
c) EDTA weight part range as fractions between about 2 and about 0.5 including 2.0, 1.9, 1.8, 1.7, 1.6, 1.5, 1.4, 1.3. 1.2, 1.1, 1.0, 0.9, 0.8, 0.7, 0.6 or 0.5 or any fractional variation thereof.

Fractional number weight parts ratios between these primary ratios are also included.

Preferably, the EDDS weight part range is about 4 to about 1.5; more preferably about 4 to about 2.

Preferably, the MGDA weight part range is about 3 to about 0.75; more preferably about 3 to about 1.

Preferably, the EDTA weight part range is about 2 to about 0.75, more preferably about 1.5 to about 0.5.

The pairing of the weight part ranges for EDDS, MGDA and EDTA to provide EDDS to MGDA to EDTA ratios may be made any order. In other words, each of the fractional parts for one of EDDS, MGDA and EDTA of the foregoing individual ranges a), b) and c) may be paired with any of the fractional parts for each of the other two chelates. For example, EDDS at 4.0 parts may be paired with MGDA at 3.0 parts and EDTA at 2.0 parts, or may be paired with MGDA at 1.0 parts and EDTA at 0.5 parts, or may be paired with MGDA at 2.0 parts and EDTA at 2.0 parts. Similarly EDDS at 2.1 parts may be paired with MGDA at 2.2 parts and EDTA at 1.5 parts or EDDS at 3.0 parts may be paired with MGDA at 1.0 parts and EDTA at 0.5 parts. The pairing of weight parts in any order provides examples of coverage of chelate mixture ratios in whole number weight part ratios for EDDS to MGDA to EDTA such as 4:1:0.5, 4:2:0.5, 4:3:0.5, 3:1:0.5, 3:2:0.5, 3:3:0.5, 2:1:0.5, 2:2:0.5 or 2:3:0.5; or 4:1:1, 4:2:1, 4:3:1, 3:1:1, 3:2:1, 3:3:1, 2:1:1, 2:2:1 or 2:3:1;or 4:1:2, 4:2:2, 4:3:2, 3:1:2, 3:2:2, 3:3:2, 2:1:2, 2:2:2 or 2:3:2. Another example of the pairing in any order using fractional weight parts provides fractional weight part ratios of EDDS to MGDA to EDTA such as 4:1.5:1, 4:2.5:1, 3:1.5:1, 3:2.5:1, 1:1.5:2, 1:2.5:2 as well as 3.5:1:0.5, 2.5:1:0.5, 3.5:2.5:0.5, 2.5:2.5:0.5, 2:2.5:0.5.

The pairing in any order works out in practical terms to provide gram weight fractions of the weights for the EDDS to MGDA to EDTA chelate mixture. For example, a 12 gm of chelate mixture:
a) may contain 6.86 gm EDDS, 1.71 gm MGDA and 3.43 gm EDTA (4:1:2 ratio), or
b) may contain 6 gm EDDS, 4 gm MGDA and 2 gm EDTA (3:2:1 ratio), or
c) may contain 4.36 gm EDDS, 6.55 gm MGDA and 1.09 gm EDTA (2:3:0.5 ratio).

The concentration of the chelate mixture in embodiments of the bleach composition is calculated to deliver from about 2 wt% to about 10 wt%, preferably about 3 wt% to about 8 wt%, more preferably up to about 6 wt%, most preferably up to about 5 wt. % of the chelate mixture when the bleach composition is diluted with water and/or developer to form an oxidizing liquid, paste or gel. To accomplish this delivery the concentration of the chelate mixture in the undiluted bleach composition comprises from about 4 wt% to about 16 wt%, preferably about 8 wt% to about 16 wt% relative to the total weight of the bleach composition.

The oxidizing agent of embodiments of the bleach composition may comprise at least one or more peroxo inorganic compounds. The one or more peroxo inorganic compounds include but are not limited to a peroxide salt, persulfate salt, a percarbonate salt, a perbicarbonate salt, a perborate salt, a hypochlorite salt, a chlorite salt, a hypobromite salt, bromite salt or any combination thereof and the cation of the salt is sodium, potassium or ammonium or any combination thereof. Especially preferred inorganic peroxo compounds include the persulfate, percarbonate/perbicarbonate and perborate salts with sodium and/or potassium cations.

The oxidizing agent of embodiments of the bleach composition may also comprise one or more organic peroxo compounds alone or in combination with one or more peroxo inorganic compounds. The one or more organic peroxo compounds include benzoyl peroxide, peroxybenzoic acid and halo derivatives thereof, pyridinium chlorochromate, peracetic acid, trifluoroacetic peracid, phthaloyl peroxide, t-butyl peroxybenzoate, t-butyl hydroperoxide, dicumyl peroxide, methyl ethyl ketone peroxide, methyl isobutyl ketone peroxide, acetone peroxide, acetyl benzoyl peroxide, di-(1-naphthoyl) peroxide, diacetyl peroxide. Preferred are solid forms of the organic peroxo compounds.

The oxidizing agent of embodiments of the bleach composition preferably comprises one or more inorganic peroxo compounds.

The amount of oxidizing agent in embodiments of the bleach composition is calculated to provide from about 3 wt% to about 30 wt% of active oxygen oxidizer when the bleach composition is diluted with water and/or with a developer such as aqueous hydrogen peroxide. To accomplish this delivery, the total concentration of the one or more inorganic peroxo compounds as the oxidizing agent in embodiments of the bleach composition comprises from about 20 wt% to about 90 wt%, preferably from about 40 wt% to about 90 wt% relative to the total weight of embodiments of the bleach composition. The total concentration of the one or more organic peroxo compounds as the oxidizing agent in embodiments of the bleach composition comprises from about 40 wt% to about 90 wt%, preferably from about 60 wt% to about 90 wt% relative to the total weight of the embodiments of the bleach composition. The total concentration of a combination of one or more inorganic and organic peroxo compounds as the oxidizing agent in embodiments of the bleach composition comprises from about 20 wt% to about 90 wt% with the portion of one or more inorganic peroxo compounds preferably being from about 60 wt% to about 95 wt% of the total concentration of the combination of inorganic and organic peroxo compounds.

The alkalizing agent is capable of establishing a basic pH of from about 8 to about 11, preferably about 8.5 to about 9.5 when embodiments of the bleach composition are combined with water and/or developer. To accomplish this basic pH, the alkalizing agent in embodiments of the bleach composition comprises an alkaline salt and/or an alkaline buffering salt. In particular, the alkalizing agent preferably comprises an alkaline metal salt, magnesium salt and/or ammonium salt of hydroxide, carbonate, bicarbonate, silicate, aluminosilicate, metasilicate, phosphate and any combination thereof. Preferred alkaline metal cations include sodium, potassium and/or lithium. Preferred salt anions include hydroxide, carbonate, silicate, hydrated silicate and metasilicate, especially mixtures of hydroxide with one or more of the buffering salt anions such as but not limited to carbonate and phosphate.

The concentration of the alkalizing agent in embodiments of the bleach composition comprises from about 10 wt% to about 60 wt%, preferably about 10 wt% to about 50 wt%, more preferably about 10 wt.% to about 40 wt.% relative to the total weight of embodiments of the bleach composition.

Embodiments of the bleach composition may have a physical form of a solid or a paste. Preferably, the physical form is a solid as a particulate or powder.

The physical form of the bleach composition may be a paste formed by combining the solid form with a fatty substance such as high weight hydrocarbons, for example mineral oil or paraffin oil and/or a C16 up to a C24 carbon count fatty alcohol and/or a C16 up to a C24 carbon count fatty acid. These fatty substances are described in detail below as fatty substance additives.

Embodiments of the bleach composition may further comprise one or more optional additives combined with the primary components of the oxidizing agent, the chelate mixture and the alkalizing agent. Any ordinary additive for a hair bleach may be included provided that the additive does not cause embodiments of the bleach composition to lose the oxidation capability of the oxidizing agent.

The one or more optional additives for embodiments of the bleach composition may include but are not limited to one or more of a hair conditioner, a fatty substance, a synthetic or natural thickener, a natural or non-natural amino acid, a detergent, e.g, an anionic surfactant; a non-detergent surfactant including one or more of a nonionic, cationic, zwitterionic and/or amphoteric surfactant; a filler, an organic polymer, an organic and/or inorganic acid, an ancillary binder, an anticaking agent, a lubricant, a disintegrant, a silicone, an amodimethicone, a polydimethylsiloxane, a rheology modifier, non-reactive organic solvent, as well as other common bleach ancillary additives.

When one or more additives are included, the concentration of the one or more additives in embodiments of the bleach composition comprises the remainder of 100 wt% minus the weight percentages of the oxidizing agent, the chelate mixture and the alkalizing agent. With inclusion of one or more additives, the chelate mixture concentration is maintained at about 4 wt% to about 16 wt%, preferably 8 wt.% to about 16 wt.% by two adjustments. First is inclusion of additional chelate mixture to embodiments of the bleach composition to maintain the weight percentage range of the chelate mixture. Second is the adjustment of the concentrations of the oxidizing agent and the alkalizing agent.

### The hair conditioner

The optional hair conditioner additive for embodiments of the bleach composition may comprise a cationic conditioning polymer or an amphoteric conditioning polymer. The cationic conditioning polymer comprises a polymer having multiple cationic groups or groups that can be ionized into cationic groups, and which can improve the cosmetic properties of keratin fibers, in particular, the disentangling, the softness, the sheen and the volume. The cationic conditioning polymers that are suitable are advantageously chosen from those already known as improving the cosmetic properties of the hair, such as but not limited to those described in patents and patent applications such as EP 337 354 and FR 2 270 846. Examples of cationic conditioning polymers include cationic polymers comprising at least primary, secondary, tertiary and/or quaternary amine groups, which either may form part of the main polymer chain, or may be borne by a side substituent directly attached thereto.

Exemplary cationic conditioning polymers include the following groups:
1) copolymers of acrylamide and of dimethylaminoethyl methacrylate quaternized with dimethylsulphate or with a dimethylhalide (Hercofloc from Hercules); copolymers of acrylamide and of methacryloyloxyethyltrimethylammonium chloride (Bina Quat P 100 from Ciba Geigy); the copolymer of acrylamide and of methacryloyloxyethyltrimethylammonium methosulphate (Reten from Hercules); quaternized or non-quaternized vinylpyrrolidone/dialkylaminoalkyl acrylate or methacrylate copolymers (Gafquat range from ISP; Copolymer 845, 958 and 937 from ISP); dimethylaminoethyl methacrylate/vinylcaprolactam/vinylpyrrolidone terpolymers (Gaffix VC 713 from ISP); vinylpyrrolidone/methacrylamidopropyldimethylamine copolymers (Styleze CC 10 from ISP); vinylpyrrolidone/dimethylaminopropylmethacrylamide quaternized copolymers (Gafquat HS 100 from ISP);
2) Cellulose ether derivatives comprising quaternary ammonium groups. These polymers are also defined in the CTFA dictionary as quaternary ammoniums of hydroxyethylcellulose that has reacted with an epoxide substituted with a trimethylammonium group;
3) Cationic cellulose derivatives such as copolymers of cellulose or cellulose derivatives grafted with a water-soluble quaternary ammonium monomer, such as hydroxyalkylcelluloses, for instance hydroxymethylcellulose, hydroxyethylcellulose or hydroxypropylcellulose grafted especially with a methacryloylethyltrimethylammonium, methacrylamidopropyltrimethylammonium or dimethyldiallylammonium salt;
4) The cationic polysaccharides such as guar gums containing trialkylammonium cationic groups; Guar gums modified with a salt, for instance the chloride, especially 2,3-epoxypropyltrimethylammonium chloride;
5) Polymers consisting of piperazinyl units and of divalent alkylene or hydroxyalkylene radicals containing straight or branched chains, optionally interrupted with oxygen, sulphur or nitrogen atoms or with aromatic or heterocyclic groups, and also the oxidation and/or quaternization products of these polymers. (6) Water-soluble polyaminoamides prepared in particular by polycondensation of an acidic compound with a polyamine, which are optionally crosslinked, optionally alkylated, or, if they comprise one or more tertiary amine functions, quaternized;
6) Polyaminoamide derivatives resulting from the condensation of polyalkylene polyamines with polycarboxylic acids followed by an alkylation with difunctional agents. Examples that may be mentioned include adipic acid-dialkylaminohydroxyalkyldialkylenetriamine polymers in which the alkyl radical is C₁-C₄;
7) Polymers obtained by reacting a polyalkylene polyamine comprising two primary amine groups and at least one secondary amine group with a dicarboxylic acid chosen from diglycolic acid and saturated C₃-C₈ aliphatic dicarboxylic acids, and then with epichlorohydrin;
8) Cyclopolymers of alkyldiallylamine or of dialkyldiallylammonium, in homopolymer or copolymer form;
9) Diquaternary ammonium polymers;
10) Quaternary polymers of vinylpyrrolidone and of vinylimidazole;
11) Polyamines of the polyethylene glycol (15) Tallow polyamine type (CTFA dictionary name);
12) Crosslinked methacryloyloxy(C₁-C₄)alkyltri(C₁-C₄)alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide. A crosslinked acrylamide/methacryloyloxyethyltrimethylammonium chloride copolymer (20/80 by weight) in the form of a dispersion containing 50% by weight of said copolymer in mineral oil (Salcare SC 92 from Ciba) can be used more particularly. A crosslinked methacryloyloxyethyltrimethylammonium chloride homopolymer containing about 50% by weight of the homopolymer in mineral oil or in a liquid;
13) Additional cationic conditioning polymers that can be used in the context of the invention are polyalkyleneimines, in particular polyethyleneimines, polymers containing vinylpyridine or vinylpyridinium units, condensates of polyamines and of epichlorohydrin, quaternary polyureylenes and chitin derivatives.

The amphoteric conditioning polymer(s) for embodiments of the bleach composition may comprise polymers have balance multiple numbers of cationic and anionic groups such as but not limited to quaternary ammonium groups and anionic carboxyl or sulfonic groups. The amphoteric conditioning polymer may be polyolefins with pendant cationic and anionic groups randomly distributed in the polymer chain, in which cationic group is derived from quaternization of a pendant amine containing olefinic monomer and the anionic group is derived from an acidic olefinic monomer comprising one or more carboxylic or sulphonic group. Alternatively, the anionic and cationic groups may be derived from zwitterionic carboxybetaine or sulphobetaine monomers; or may be a cationic polymer chain comprising primary, secondary, tertiary or quaternary amine groups, in which at least one of the amine groups bears a carboxylic or sulphonic group linked via a hydrocarbon-based radical; or may be part of a chain of a polymer containing an α,β-dicarboxylic ethylene unit in which one of the carboxylic groups has reacted with a polyamine comprising one or more primary or secondary amine groups. Exemplary amphoteric conditioning polymers include:
1) polymers resulting from the copolymerization of a monomer derived from a vinyl compound bearing a carboxylic group such as, more particularly, (meth)acrylic acid, maleic acid, α-chloroacrylic acid, or else a dialkyldiallylammonium salt such as dimethyldiallylammonium chloride, and a basic monomer derived from a substituted vinyl compound containing at least one basic atom, such as, more particularly, dialkylaminoalkyl methacrylate and acrylate, dialkylaminoalkylmethacrylamide and - acrylamide, as described in U.S. Pat. No. 3,836,537. Mention may also be made of the sodium acrylate/acrylamidopropyltrimethylammonium chloride copolymer (Polyquart KE 3033 from Cognis) and the acrylic acid/dimethyldiallylammonium chloride copolymer (Merquat 280, 295, Plus 3330, from Nalco);
2) polymers containing units derived from a) at least one monomer chosen from (meth)acrylamides substituted on the nitrogen with an alkyl radical, in particular C₂-C₁₂, b) at least one acidic monomer containing one or more reactive carboxylic groups (for example (meth)acrylic acid, crotonic acid or itaconic acid, and monoesters of maleic or fumaric acids or anhydrides), and c) at least one basic monomer such as esters containing primary, secondary, tertiary and quaternary amine substituents of (meth)acrylic acid, fumaric acid or maleic acid, and the product of quaternization of dimethylaminoethyl methacrylate with dimethyl or diethyl sulphate;
3) Octylacrylamide/acrylate/butylaminoethyl methacrylate copolymers (Amphomer or Lovocryl 47 by National Starch);
4) Crosslinked and partially or totally alkylated polyaminoamides, derived from polyaminoamides of general formula -[CO-R⁵-CO-Z]- in which R⁵ is a divalent radical derived from a saturated or unsaturated dicarboxylic acid (for example adipic acid, 2,2,4-trimethyladipic acid, 2,4,4-trimethyladipic acid, terephthalic acid or itaconic acid), from an unsaturated monocarboxylic acid (for instance (meth)acrylic acid), from a C₁-C₆ alkyl ester of the abovementioned acids or from a radical derived from the addition of one of these acids to a bis-primary or bis-secondary amine, and Z denotes a radical of a bis-primary, mono- or bis-secondary polyalkylene-polyamine. Preferably, Z represents between 60 and 100 mol %, the radical -NH-[(CH₂)ₓ-NH]ₚ- with x=2 and p=2 or 3, or x=3 and p=2; this radical is derived from diethylenetriamine, from triethylenetetramine or from dipropylenetriamine; between 0 and 40 mol % the above radical, in which x=2 and p=1 and which is derived from ethylenediamine, or the radical derived from piperazine -N[CH₂CH₂]₂N-; between 0 and 20 mol %, the radical -NH-(CH₂)₆-NH- derived from hexamethylenediamine. The crosslinking agent for these polymers is a difunctional agent chosen from epihalohydrins, diepoxides, dianhydrides and bis-unsaturated derivatives, and alkylated by the action of acrylic acid, chloroacetic acid or an alkane sultone or the alkali metal salts thereof;
5) Polymers comprising at least zwitterionic units, for instance the butyl methacrylate/dimethylcarboxymethylammonioethyl methacrylate copolymer (Diaformer Z301 from Sandoz).

The conditioner additive for embodiments of the bleach composition may also comprise a cationic surfactant comprising a lipophilic tail and a quaternary ammonium head wherein the nitrogen is quaternized with four organic groups. Cationic surfactants useful herein include, for example, those corresponding to the general formula: 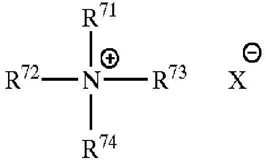 wherein at least one of R71, R72, R73 and R74 is selected from an aliphatic group of from about 8 to about 30 carbon atoms or an aromatic, alkoxy, polyoxyalkylene, alkylamido, hydroxyalkyl, aryl or alkylaryl group having up to about 22 carbon atoms, the remainder of R⁷¹, R⁷², R⁷³ and R⁷⁴ are independently selected from an aliphatic group of from about 1 to about 22 carbon atoms or an aromatic, alkoxy, polyoxyalkylene, alkylamido, hydroxyalkyl, aryl or alkylaryl group having up to about 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether, ester, or amido linkages and other groups such as amino groups. The longer chain aliphatic groups, (e.g., those of about 12 carbons, or higher), can be saturated, unsaturated, or branched. Preferred is when R71, R72, R73 and R74 are independently selected from C1 to about C22 alkyl. Nonlimiting examples of cationic surfactants useful in the present invention include the materials having the following CTFA designations: quaternium-8, quaternium-14, quaternium-18, quaternium-18 methosulfate, quaternium-24, and mixtures thereof.

Among the cationic surfactants of general formula above, preferred are those containing in the molecule at least one alkyl chain having at least 16 carbons. Nonlimiting examples of such preferred cationic surfactants include: behenyl trimethyl ammonium chloride available, for example, with tradename Genamine KDMP from Clariant, with tradename Incroquat Behenyl TMS-50 from Croda, and with tradename ECONOL TM22 from Sanyo Kasei; cetyl trimethyl ammonium chloride available, for example, with tradename CTAC 30KC from KCI, and with tradename CA-2350 from Nikko Chemicals; stearyl trimethyl ammonium chloride available from Parchem; olealkonium chloride available, for example, with tradename Incroquat ^{™} TMS-50 from Croda; hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methyl) pyridinium chloride.

Also preferred are hydrophilically substituted cationic surfactants in which at least one of the substituents contains one or more aromatic, ether, ester, amido, or amino moieties present as substituents or as linkages in the radical chain, wherein at least one of the R71-R74 radicals contain one or more hydrophilic moieties selected from alkoxy (preferably C1-C3 alkoxy), polyoxyalkylene (preferably C -C3 polyoxyalkylene), alkylamido, hydroxyalkyl, alkylester, and combinations thereof. Preferably, the hydrophilically substituted cationic conditioning surfactant contains from about 2 to about 10 nonionic hydrophile moieties located within the above stated ranges. Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commercially available under the following tradenames; VARISOFT 110, VARISOFT 222, VARIQUAT K1215 and VARIQUAT 638 from Evonik; MACKPRO KLP, MACKPRO WLW, MACKPRO MLP, MACKPRO NSP, MACKPRO NLW, MACKPRO WWP, MACKPRO NLP, MACKPRO SLP from Solvay; ETHOQUAD 18/25, ETHOQUAD O/12PG, ETHOQUAD C/25, ETHOQUAD S/25, and ETHODUOQUAD from Akzo; DEHYQUART SP from BASF; and ATLAS G265 from Croda. Babassuamidopropalkonium Chloride available from Croda under the tradename Incroquat BA-85 is also preferably used in the composition.

While a hair conditioner can be included in bleach composition embodiments of the invention, preferred embodiments of the bleach composition are substantially free to essentially free of hair conditioner. Typically a hair conditioner is separately applied to the hair following bleaching so that the alkaline pH delivered by the bleaching process is converted to at least neutral and preferably slightly acidic. If present, the concentration of the hair conditioner in the bleach composition may be in a range of from about 0.01 wt.% to about 10 wt.%, preferably up to about 5 wt.%, more preferably up to about 1 or 2 wt.% relative to the total weight of the bleach composition.

### The Fatty Substance Additive

The fatty substance additive for embodiments of the bleach composition includes moderate to high melting functionalized fatty compounds with a terminal alcohol group, a carboxylic acid group, an ester group or an amine group. The "fatty" moiety of the fatty compound comprises a C 8-C40 linear or branched, saturated alkyl group. Included are fatty alcohols, fatty acids, fatty esters and fatty amines. The fatty substance as an additive also includes nonfunctionalized fatty compounds such as C10 -C40 linear or branched, saturated hydrocarbons.

Preferred fatty substances include fatty alcohols and hydrocarbons. Preferred embodiments of the bleach composition may include either or both of these two fatty substances. A preferred a fatty alcohol and/or hydrocarbon may each be present in embodiments of the bleach composition at a concentration of from about 0.1 wt% to about 10 wt%, preferably from about 0.1 wt% to about 5 wt%, more preferably from about 0.1 wt% to about 2 wt%.

### The Fatty Alcohol, Acid and/or Amine Additive

The functionalized fatty compound additive for embodiments of the bleach composition may comprise a fatty alcohol or derivative thereof, a fatty acid or derivative thereof a fatty amine or derivative thereof. This moderate to high melting fatty compound has the general formula R-X, wherein R is an aliphatic (e.g. fatty chain) and X is a functional group (e.g. alcohol, carboxylic acid, amine or derivative). The aliphatic group R may be a linear or branched saturated carbon chain of 8 to 40 carbons, preferably 12 to 30 carbons. The fatty compound is selected from the group consisting of fatty alcohols, fatty acids, fatty alcohol derivatives, fatty acid derivatives, fatty amine, fatty amine derivatives and mixtures thereof. Preferred melting points for the moderate to high melting fatty compounds span a range of at least 0°C with no upper limit. The melting point preferably is sufficiently above the freezing point of water to avoid causing embodiments of the solid bleach composition to adopt a physical character of a "mushy sludge" when more than a minimal concentration of the fatty compound is present in these embodiments. Having a physical character of a "mushy sludge" can prevent accurate measurement of portions of embodiments of the bleach composition. Nonlimiting examples of the moderate to high melting point compounds are found in International Cosmetic Ingredient Dictionary, Fifth Edition, 1993, and CTFA Cosmetic Ingredient Handbook, Second Edition, 1992.

The fatty alcohols, fatty acids, fatty amines and fatty alcohol esters are capable of functioning in embodiments of the bleach composition as binders for formation of substantially uniform solid particles formed from nonuniform dust of bleach composition, dust suppression material and dispersants for water insoluble additives.

The fatty alcohols useful herein are those having from about 8 to about 40 carbon atoms, preferably from about 16 to about 30, more preferably up to 22 carbon atoms. These fatty alcohols are saturated and can be straight or branched chain alcohols. Nonlimiting examples of fatty alcohols include decanol, undecyl alcohol, lauryl alcohol, tridecyl alcohol, myristyl alcohol, pentadecyl alcohol, cetyl alcohol, palmityl alcohol, heptadecyl alcohol, stearyl alcohol, saturated oleyl alcohol, nonadecyl alcohol, arachidyl alcohol, heneicosyl alcohol, behenyl alcohol, saturated erucyl alcohol, lignoceryl alcohol, ceryl alcohol, heptacosanol, montanyl alcohol, nonacosnol, myricyl alcohol, dotriacontanol, geddyl alcohol and mixtures thereof. Preferred are lauryl alcohol, myristyl alcohol, cetyl alcohol, stearyl alcohol, saturated oleyl alcohol, arachidyl alcohol, behenyl alcohol, saturated erucyl alcohol and mixtures thereof such as cetearyl alcohol (mixture of cetyl and stearyl alcohols).

The fatty acids useful herein are those having from about 10 to about 30 carbon atoms, preferably from about 12 to about 36 carbon atoms, and more preferably from about 16 to about 30 carbon atoms. These fatty acids are saturated and can be straight or branched chain acids. Also included are diacids, triacids, and other multiple acids which meet the requirements herein. Also included herein are alkaline, magnesium and ammonium salts and especially the sodium, potassium, lithium and ammonium salts of these fatty acids. Nonlimiting examples of fatty acids include caprylic acid, capric acid, lauric acid, cetylic acid, palmitic acid, stearic acid, behenic acid, sebacic acid, arachidic acid, behenic acid, saturated oleic acid, palmitolic acid, elaidic acid, linolanic acid, saturated linolenic acid, saturated arachidonic acid, saturated eicosapentaenoic acid, saturated docohexaenoic acid or mixtures thereof.

Commercially available high melting point fatty alcohols and acids useful herein include: cetyl alcohol, stearyl alcohol, and behenyl alcohol available from Parchen, various fatty acids having tradenames NEO-FAT available from Akzo, HYSTRENE available from PMC Group.

Fatty amines include primary, secondary, and tertiary fatty amines are useful. Particularly useful are amines having an alkyl group of from about 12 to about 30 carbons. Exemplary amines include: stearylamine, stearyldiethylamine, stearyldimethylamine, palmityl amine, palmityldiethylamine, palmityldimethylamine, behenylamine, behenyldiethylamine, behenyldimethylamine, arachnidylamine, arachidyldiethylamine, arachidyldimethylamine, erucyl amine, lignoceryl amine, ceryl amine, montanyl amine, muricyl amine, and linear alkenyl amines wherein the olefinic groups are hydrogenated to saturation. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidylbehenylamine.

The fatty alcohol derivatives may be mono alcohols, polyols, polyethers, polyepoxys, esterified fatty alcohols, esterified polyols, esterified polyethers and mixtures thereof. The fatty alcohol derivatives and fatty acid derivatives useful herein include alkyl ethers of fatty alcohols, alkoxylated fatty alcohols, alkyl ethers of alkoxylated fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth-10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, (e.g. a mixture of fatty alcohols containing predominantly cetyl and stearyl alcohol, wherein the numeric designation indicates the number of ethylene glycol moieties present); C1-C30 alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

The fatty alcohol ester is composed of an esterified acid which is a monocarboxylic acid of 3 to 10 carbons such as acetic, propionic, butyric, valeric, caproic, enanthic, caprylic pelargonic, capric acid or the linear dicarboxylic acid of 3 to 10 carbons without hydroxyl as described above for the primary component. The fatty alcohol is the fatty alcohol of 10 to 30 carbons as described above. The dicarboxylic acid for the fatty alcohol ester version includes the above described preferred and more preferred versions thereof also. The fatty alcohol may be saturated or hydrogenated versions of unsaturated fatty alcohols including such fatty alcohols as lauryl, tridecyl, myristyl, penatadecyl cetyl, palmitoleyl, heptadecyl, stearyl, oleyl, nonadecyl, arachidyl, heneicosyl, behenyl, erucyl, lignoceryl, ceryl, heptacosanyl, montanyl, nonacosanyl and myricyl alcohol wherein the unsaturated fatty alcohols such as palmitoleyl, oleyl, heneicosyl and erucyl alcohol are hydrogenated. The carboxyl variation of the acid provides at least three versions of the ester. These are a monocarboxylic acid esterified with a fatty alcohol, a dicarboxylic acid esterified once with a fatty alcohol and a dicarboxylic acid esterified twice with a fatty alcohol. Additionally, the lipid character of the single esterification of the dicarboxylic acid may be enhanced by esterifying the remaining carboxyl group with a monoalcohol of 1 to 6 carbons.

The fatty acid ester is composed of a fatty acid as described above which is esterified with a C1-C6 mono alcohol or with a C2-C6 diol or a C3-C6 triol. The mono, di and tri esterification of a triol such as glycerin or trihydroxy butane, pentane or hexane provides a substantially to essentially water insoluble fatty ester that can function along with a detergent additive to solubilize and/or disperse water insoluble additives of the bleach composition.

### The Hydrocarbon Additive

The hydrocarbon or hydrocarbon mimic can function as a dust controller and dust/microparticle binding agent for embodiments of the bleach composition. The hydrocarbon or hydrocarbon mimic comprises a saturated linear or branched hydrocarbon chain ranging in carbon count from 10 to 40 carbons, preferably 20 to 40 carbons. Preferably, the hydrocarbon is branched with methyl groups pendant to the hydrocarbon chain.

Exemplary hydrocarbons include liquid petroleum comprising a mixture of linear and/or branched C10 - C30 hydrocarbon chains. Additional exemplary hydrocarbons include saturated and hydrogenated unsaturated mono-terpenes (C10) di-terpenes (C20), tri-terpenes (C30) sesqui versions thereof (C15, C25) and exemplary terpenes such as 2,6-dimethyl octane, 2,6-dimethyl octene, 2-methyl-6-ethyl heptane, 2-methyl-6-ethyl heptene, α- and/or β- farnesane, isofarnesane, neofarnesane, farnesene, isofarnesene, neofarnesene, pristane, isopristane, neopristane, pristene, isopristene, neopristene, phytane isophytane, neophytane, phytene, isophytene, neophytene, squalane, isosqualane, neosqualane, squalene, isosqualene, neosqualene or any combination thereof as well as liquid hydrogenated polyisoprene, isoparaffin, mineral oil and liquid olefin polymer wherein the hydrocarbon polymer is liquid at standard pressure and temperature.

The preferred hydrocarbon may be squalane or a pendant multi-methyl substituted C24-C30 saturated hydrocarbon chain or mixtures thereof.

Exemplary hydrocarbon mimics include silicone oil, natural oils such as olive oil, soybean oil, corn oil, coconut oil, rape seed oil, flax seed oil, argan oil, jojoba oil and passion fruit oil.

Additional alternative hydrocarbon additives include vegetable oils such as but not limited to soybean oil, corn oil, palm oil, rape seed oil, peanut oil, coconut oil, olive oil, almond oil, cashew oil, canola oil and others; preferably purified to separate remove fatty acid residues for optional use as the fatty acid feature of the fatty substance described above.

### The Rheology Modifier and Thickener Additive

A "rheology modifier" is substantially synonymous with a thickener and is a compound capable establishing a thickening viscosity for embodiments of the bleach composition when diluted with an aqueous medium to provide a semi-fluid consistency such that it does not run, and remains localized when applied to hair. The rheology modifier/thickener additive is typically water soluble and may be present in embodiments of the bleach composition at concentration ranges from about 0.05 wt% to about 5 wt%.

The rheology modifier, a.k.a. thickener, comprises a natural or synthetic polymer for use in cosmetics that swells, thickens, and/or interacts electrostatically and/or by hydrogen bonding with an aqueous medium containing hydroxyl and amine compounds such as monoalcohols, glycerin, organic acids and bases to increase the viscosity of the medium to have a viscosity at least like whole milk and preferably like the viscosity of molasses at 10°C to 15°C.

The rheology modifier/thickener additive as one or more synthetic and natural polymers includes at least polyolefins, polyesters, polyamides, polyurethanes, polyureas polysaccharides, polyaminoacids and copolymers thereof . The synthetic and natural polymer at least in part contributes to rheology control and thickening of embodiments of the bleach composition when the embodiments are diluted and/or combined with an aqueous medium.

Included are homopolymers and copolymers containing ethylenically unsaturated monomers of the ester and/or amide type, as well as hydroxy alkyl ester monomers, diacid/diamine monomers and the like to form a polyester and/or a polyamide optionally and preferably with ester and/or amide pendant groups. Included are a homo- and copolymer of vinylpyrrolidone, polysaccharides, polyacrylamides, methyl methacrylate/ethylene glycol dimethacrylate copolymers, butyl methacrylate/methyl methacrylate copolymers, and polymethyl methacrylates, glucans, modified or unmodified starches (i.e. hydroxypropyl starch phosphate), amylose, amylopectin, glycogen, dextrans, celluloses and derivatives thereof (methylcelluloses, hydroxyalkylcelluloses, ethylhydroxyethylcelluloses), mannans, xylans, lignins, arabans, galactans, galacturonans, chitin, chitosans, glucoronoxylans, arabinoxylans, xyloglucans, glucomannans, pectic acids and pectins, arabinogalactans, carrageenans, agars, gums arabic, gums Tragacanth, Ghatti gums, Karaya gums, xanthan, acacia gum, locust bean, guar, agar, carob gums, galactomannans and mixtures thereof.

The synthetic polymers include but are not limited to functionalized olefinic polymers, polyamides, polyols, polyesters, polyethers optionally having pendant functional groups such as carboxylic acid, alkylenyl amine, alkylenyl alcohol and mixtures thereof. Exemplary rheology modifier/thickener synthetic polymers include polyvinylpyrrolidone, polyacrylic acid, polyacrylamide, polyethylene oxide, polypropylene oxide, polyvinyl acetate, polyvinyl pyrrolidone, polyacrylamide, polyoxyethylene-C10-C20 alkyl ether non-crosslinked poly-2-acrylamidopropanesulfonic acid, acid crosslinked poly-2-acrylamido-2-methylpropane sulfonic acid, cross-linked poly-2-acrylamido-2-methylpropane sulfonic acid partially neutralized by ammonia. non-crosslinked poly-2-acrylamido-2-methylpropane sulfonic acid in combination with hydroxyalkylcellulose ethers or with poly (ethylene oxide). and similar polymers non-crosslinked poly(2-acrylamidopropanesulphonic acid) (Simugel EG from the company SEPPIC), crosslinked poly(2-acrylamido-2-methylpropanesulphonic acid), free or partially neutralized with ammonia (Hostacerin AMPS from Clariant), mixtures of non-crosslinked poly(2-acrylamido-2-methylpropanesulphonic acid) with hydroxyalkylcellulose ethers or with poly(ethylene oxide), mixtures of poly((meth)acrylamido(C₁-C₄)alkylsulphonic acid), which is preferably crosslinked, with a crosslinked copolymer of maleic anhydride and of a (C₁-C₅)alkyl vinyl ether (Hostacerin AMPS/Stabileze QM from the company ISF).

The thickening polymers of natural origin are preferably polymers comprising at least one sugar unit, which comprise a monosaccharide (i.e. monosaccharide or oside or simple sugar) portion, an oligosaccharide portion (short chains formed from a sequence of monosaccharide units, which may be different) or a polysaccharide portion [long chains consisting of monosaccharide units, which may be different, i.e. polyholosides or polyosides]. The saccharide units may also be substituted with alkyl, hydroxyalkyl, alkoxy, acyloxy or carboxyl radicals, the alkyl radicals containing from 1 to 4 carbon atoms.

Exemplary natural sugar unit polymers include but are not limited to nonionic guar gums, optionally modified with C₁C₆ hydroxyalkyl groups; biopolysaccharide gums of microbial origin, such as scleroglucan gum or xanthan gum; gums derived from plant exudates, such as gum arabic, ghatti gum, karaya gum, gum tragacanth, carrageenan gum, agar gum and carob gum; pectins; alginates; starches including such examples as rice starch, corn starch, potato starch, canola starch, cassava starch and hydrolyzed derivatives thereof; hydroxy (C₁-C₆) alkylcelluloses, carboxy(C₁-C₆) alkylcelluloses, non-ionic guar gums, semisynthetic guar gums modified with C1-C6 hydroxylalkyl groups; biopolysaccharide gums of microbial origin such as Scleroglucan or xanthan gums.

Examples of nonionic, unmodified guar gums include Guargel D/15 (Goodrich); Vidogum GH 175 (Unipectine), Meypro-Guar 50 and Jaguar C (Meyhall/Rhodia Chimie); and the modified nonionic guar gums that may be mentioned include Jaguar HP8, HP60, HP120, DC 293 and HP 105 (Meyhall/Rhodia Chimie); Galactasol 4H4FD2 (Aqualon).

Examples of cellulosic thickeners include but are not limited to of water soluble cellulosic thickeners include but are not limited to hydroxyalkyl (C ₁ -C ₆ ) celluloses such as hydroxyethylcelluloses such as those trademarked as CELLOSIZE and hydroxypropylcelluloses such as those trademarked as KLUCEL. Additional embodiments of cellulosic thickeners include but are not limited to carboxyalkyl (C ₁ -C ₆ ) celluloses such as carboxymethylcellulose trademarked as BLANOSE, AQUASORB, AMBERGUM and CELLOGEN.

Further examples of gums include scleroglucans such as, especially, Actigum CS from Sanofi Bio Industries; Amigel from Alban Muller International, and also the glyoxal-treated scleroglucans described in FR 2 633 940); xanthan gums, for instance Keltrol, Keltrol T, Keltrol Tf, Keltrol Bt, Keltrol Rd, Keltrol Cg (Nutrasweet Kelco), Rhodicare S and Rhodicare H (Rhodia Chimie); starch derivatives, for instance Primogel (Avebe); hydroxyethylcelluloses such as Cellosize QP3L, QP4400H, QP30000H, HEC30000A and Polymer PCG10 (Amerchol), Natrosol 250HHR, 250MR, 250M, 250HHXR, 250HHX, 250HR, HX (Hercules) and Tylose H1000 (Hoechst); hydroxypropylcelluloses, for instance Klucel EF, H, LHF, MF and G (Aqualon); carboxymethylcelluloses, for instance Blanose 7M8/SF, refined 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello) and Primellose (Avebe).

Additional rheology modifier/thickener additives for the bleach composition include but are not limited to aluminum phyllosilicates such as kaolin clay, a smectite clay and/or a sepiolite clay. Examples of such claims include but are not limited to bentonite clay and hectorite clay. Clays of this kind are described in "Introduction to Clay Minerals", B. Velde, Springer Science & Business Media BV, 1992.

### The Anti-caking and Lubricant Additive

An anti-caking additive for embodiments of the bleach composition includes but are not limited to one or more anti-caking agents. Examples of the one or more anti-caking agents include but are not limited to silica, magnesium stearate, sodium silicate, sodium aluminosilicate, bentonite and starch. Preferable is silica, magnesium stearate and sodium silicate at an exemplary concentration of from about 0.01 wt% to about 2 wt% relative to the total weight of the bleach composition.

A lubricant additive for embodiments of the bleach composition may be a hair conditioner described above and/or a detergent and/or cationic surfactant additive described below. Because of their "slippery" nature, surfactants such as those described below, and especially the anionic surfactant alkali metal fatty acid carboxylate salts provide a lubrication function enabling solid ingredients to slip by each other during the granulation process. The lubrication function facilitates the homogeneity of aliquots of embodiments of the bleach composition according to the invention. Examples of surfactants include anionic, nonionic and cationic surfactants, preferably a carboxylic anionic surfactant.

### The Detergent Additive

The detergent additive for embodiments of the bleach composition may comprise at least one member of an anionic surfactant including a sulfate anionic surfactant or a non-sulfate anionic surfactant including sulfonates, isethionates, carboxylates and derivatives thereof. The anionic surfactant group may also include multiple species of each member as well as a combination of these group members and any combination of multiple species of both members. Preferably, the surfactant component may comprise one or more sulfate anionic surfactants alone, one or more non-sulfate anionic surfactants alone or a mixture of a one or more sulfate anionic surfactants and one or more non-sulfate anionic surfactants.

Embodiments of the bleach composition may usually include the detergent additive and the detergent additive may be included at a concentration of from about 0.1 wt% to about 10 wt%, preferably from about 0.1 wt% to about 5 wt%, more preferably from about 0.1 wt% to about 2 wt% relative to the total weight of the bleach composition.

### Sulfate Detergent Additive

The sulfate anionic surfactant group of the detergent additive for embodiments of the bleach composition may comprise a branched and/or linear C8 to C20 alkyl and/or alkenyl sulfate, a linear and/or branched C8 to C20 alkyl benzene sulfate or a linear and/or branched C8 to C20 alkyl benzyl sulfate or the corresponding ether and/or glyceryl and/or sugar alcohol derivatives obtained from ethylene epoxide/ethylene glycol, propylene epoxide/1,2-propylene glycol, glycerin and/or sugar alcohol monomers. The linear and/or branched alkyl and/or alkenyl group may be a single chain length or may be a mixture of chain lengths. The alkenyl group may have 1 or 2 sites of unsaturation. The ether and glyceryl derivatives may comprise mono, di, tri, tetra, penta monomers up to 100, preferably up to 50, more preferably up to 20 monomeric units. The sugar alcohol derivatives may comprise mono, di, tri, tetra, penta monomers up to 100, preferably up to 50, more preferably up to 20 monomeric units of a C4-C6 sugar alcohol including but not limited to examples such as erythritol, xylitol, mannitol, sorbitol, isomalt and maltitol. The counterion of the sulfate anionic surfactant may be an alkali metal, ammonia and/or a primary, secondary or tertiary C1-C6 alkyl amine or aminoalcohol such as but not limited to ammonium cation, sodium, potassium, trimethyl amine, triethyl amine, ethanolamine, triethanolamine, diethanolamine.

The sulfate anionic surfactant may be represented by the chemical formulas: ROSO₃M, RC₆H₄OSO₃M, RC₆H₄CH₂OSO₃M, RO(C₂H₄O)ₓOSO₃M, RO(C₂H₄O)ₓC₆H₄OSO₃M, RO(C₂H₄O)ₓC₆H₄CH₂OSO₃M, RO(CH₂CH(Me)O)ₓOSO₃M, RO(CH₂CH(Me)O)ₓC₆H₄OSO₃M, RO(CH₂CH(Me)O)ₓC₆H₄CH₂OSO₃M, RO(CH₂CHOHCH₂O)ₓOSO₃M, RO(CH₂CHOHCH₂0)ₓC₆H₄OSO₃M, RO(CH₂CHOHCH₂O)ₓC₆H₄CH₂OSO₃M, RO(sugar alcohol)ₓOSO₃M, RCO₂(C₂H₄O)ₓOSO₃M, RCO₂(C₂H₄O)ₓC₆H₄OSO₃M, RCO₂(C₂H₄O)ₓC₆H₄CH₂OSO₃M, RCO₂(CH₂CH(Me)O)ₓOSO₃M, RCO₂(CH₂CH(Me)O)ₓC₆H₄OSO₃M, RCO₂(CH₂CH(Me)O)ₓC₆H₄CH₂OSO₃M, RCO₂(CH₂CHOHCH₂0)ₓOSO₃M, RCO₂(CH₂CHOHCH₂O)ₓC₆H₄OSO₃M, RCO₂(CH₂CHOHCH₂O)ₓC₆H₄CH₂OSO₃M, RCO₂(sugar alcohol)ₓOSO₃M. The R group of these formulas may be a linear or branched C8 to C20 alkyl and/or alkenyl group derived from a fatty alcohol and may be a single chain length or may be a mixture of chain lengths. The RCO₂ group is a fatty acid derivative corresponding to the fatty alcohol derivative RO-. The designator x of the ether/polyether/polyglyceryl/polysugar alcohol unit may be an integer of from 1 to 100, preferably up to 50, more preferably up to 20. Me is methyl and M is a counterion as described above. Preferred members of this class include the linear and/or branched sulfate anionic surfactants of the formulas ROSO₃M, RC₆H₄OSO₃M, RO(C₂H₄O)ₓOSO₃M and/or RO(CH₂CHOHCH₂O)ₓOSO₃M. The preferred alkyl/alkenyl group of these preferred members may be a C8 to C20 linear or branched alkyl group or a C14 to C20 linear or branched alkenyl group or mixtures of alkyl and/or alkenyl lengths or mixtures of alkyl and alkenyl groups. More preferably, the alkyl group may be a C10 to C18 linear or branched single length alkyl or mixture of lengths and the alkenyl group may be a C14 to C20 linear single length alkenyl group and/or a mixture thereof. Additionally included are mixtures of alkyl and alkenyl groups. Especially more preferably the alkyl and alkenyl group may be capryl, undecyl, lauryl, myristyl, palmityl, cetyl, stearyl oleyl, myristolyl, palmitolyl, sapienyl or mixtures thereof such as cocoyl which is a mixture of alkyl groups derived from coconut oil and includes caprylic, capric, lauric, myristic, palmitic and oleic groups.

Exemplary sulfate anionic surfactants comprise one or more of sodium lauryl sulfate, sodium laureth sulfate, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric mono glyceride sodium sulfate, sodium lauryl sulfate, sodium laureth sulfate, potassium lauryl sulfate, potassium laureth sulfate, ammonium cocoyl sulfate, sodium cocoyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfate, sodium dodecyl benzene sulfate.

### Sulfonate Detergent Additive

The non-sulfate anionic surfactant group of the detergent additive comprises a sulfonate and a sulfonate-like anionic surfactant and may be selected from the group of acyl isethionates/methyl isethionates, acyl glycinates, acyl taurates, acyl amino acids, acyl sarcosinates, sulfosuccinates and sulfonates, wherein the acyl groups of all these surfactant classes comprise from 6 to 30 carbon atoms. The non-sulfate anionic surfactant may also comprise more than one of these surfactants and may comprise mixtures thereof. The non-sulfate anionic surfactant segment may also comprise the alkaline and alkaline earth salts of one or more or mixtures of individual compounds of any of these surfactant classes and mixtures thereof.

Another non-sulfate anionic detergent additive of the bleach composition may comprise acyl isethionates/methyl isethionates. This group comprises fatty acid esters of 2-hydroxyethyl sulfonate and 2-hydroxy-2-methylethyl sulfonate. These acyl isethionates/methyl isethionates have the respective formulas RCO₂CH₂CH₂SO₃X and RCO₂CH(CH₃)CH₂SO₃X. The substituent X may be hydrogen or an alkaline or alkaline earth metal. The acyl group may be a saturated or unsaturated group RCO- of 6 to 30 carbons in which R is a linear or branched, preferably linear saturated alkyl group of 5 to 29 carbons and/or is a linear unsaturated alkyl group of 5 to 29 carbons and 1 to 3 unsaturation sites. In other words, the acyl group, RCO-, corresponds to the acyl derivative of a C6-C30 fatty acid, including but not limited to a C6-C30 fatty linear or branched, preferably linear alkyl carboxylic acid and/or a C6-C30 alkenyl carboxylic acid with 1, 2 or 3 unsaturation sites in which the carboxylic acid group of the alkyl carboxylic acid and the alkenyl carboxylic acid is positioned at a terminus.

Exemplary acyl isethionates and methyl isethionates are esters formed with isethionic acid (HOCH₂CH₂SO₂OH) and/or methyl isethionic acid and one or more C6-C30 fatty acids. Particular embodiments of the acyl group, RCO-, derived from a C6-C30 fatty acid may be selected from saturated acyl groups including but not limited to hexoyl, heptoyl, capryloyl, caproyl, lauroyl, myristoyl, palmitoyl (cetoyl), stearoyl, arachidoyl, behenoyl, lignoceroyl, cerotoyl, nonoyl, undecoyl, tridecoyl, pentadecoyl, heptadecoyl, nonadecoyl; and from unsaturated acyl groups including but not limited to myristoyl, palmitolyl, sapienoyl, oleyl, elaidoyl, vaccenoyl, linolenoyl, linoelaidoyl, arachidonoyl, eicosapentaenoyl, erucoyl, docosahexaenoyl group and any combination of saturated and/or unstaturated groups thereof. While the trivial name acyl groups are derived from natural fatty acids, the systemically nomenclature acyl groups such as nonoyl, undecoyl, tridecoyl, pentadecoly, heptadecoyl, nonadecoyl are semi-synthetic fatty acids which may be derived from petroleum sources.

Additional anionic detergent additives for embodiments of the bleach composition may comprise particular alkaline salts (sodium salts, magnesium salts, ammonium salts, amine salts, amino alcohol salts, etc.) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, α-olefin sulphonates, paraffin sulphonates; (C₆-C₂₄) alkyl sulphosuccinates, (C₆-C₂₄) alkyl ether sulphosuccinates, (C₆-C₂₄) alkylamide sulphosuccinates; (C₆-C₂₄) alkyl sulphoacetates; (C₆-C₂₄)acyl sarcosinates; and (C₆-C₂₄)acyl glutamates as well as fatty acid salts such as the salts of oleic, ricinoleic, palmitic and stearic acid, and the salts of coconut oil acid or hydrogenated coconut oil acid; acyl lactylates in which the acyl radical contains 8 to 20 carbon atoms; alkyl D-galactoside uronic acids and their salts; polyoxyalkylenated (C₆-C₂₄) alkyl ether carboxylic acids, polyoxyalkylenated (C₆-C₂₄)alkylamido ether carboxylic acids and their salts, in particular those comprising from 2 to 50 alkylene oxide groups and more, especially ethylene oxide groups, and mixtures thereof.

### Carboxylate Detergent Additive

The carboxylate detergent additive for embodiments of the bleach composition is a salt of a fatty carboxylic acid or carboxylate containing natural oil (hereinafter a carboxylate anionic surfactant) and is gentle detergent additive relative to the stronger sulfate and sulfonate detergents. The caboxylate detergent additive may be used, alone or as mixtures, and is salt of an alkaline metal including cations such as sodium, magnesium, ammonium, amine, amino alcohol.

Any of the above-described fatty acids may be employed as a carboxylate anionic surfactant by its formation as a salt with an alkaline metal, magnesium, ammonium, amine or amino alcohol cation.

Exemplary carboxylate anionic surfactants include salts such as the salts of caprylic acid, capric acid, lauric acid, myristic acid, palmitic acid, arachidinic acid, behenic acid, cerotic acid lignoceric acid, and hydrogenated oleic acid, hydrogenated ricinoleic acid, hydrogenated erucylic acid, hydrogenated cerylic acid, myricylic acid, and the salts of coconut oil acid or hydrogenated coconut oil acid; acyl lactylates in which the acyl radical contains 8 to 30 carbon atoms; alkyl D-galactoside uronic acids and their salts; polyoxyalkylenated (C₆-C₂₄)alkyl ether carboxylic acids, polyoxyalkylenated (C₆-C₂₄)alkylamido ether carboxylic acids and their salts, in particular those comprising from 2 to 50 alkylene oxide groups and more, especially ethylene oxide groups, and mixtures thereof.

Exemplary anionic carboxylic acid salts also include (C₆-C₂₄)alkylpolyglycoside carboxylic esters such as alkylpolyglucoside citrates, alkylpolyglucoside tartrates, alkylpolyglucoside sulphosuccinates and alkylpolyglucoside sulphosuccinamates;, the alkyl or acyl radical of all of these different compounds preferably containing from 12 to 20 carbon atoms and the aryl radical preferably denoting a phenyl or benzyl group.

### Non-Detergent Zwitterionic and Amphoteric Surfactant Additives

The optional surfactant(s) additive for embodiments of the bleach composition may include, without preference, nonionic, amphoteric and cationic surfactants.

Embodiments of the bleach composition may include a non-detergent surfactant at a concentration of from about 0.01 wt% to about 3 wt%, preferably from about 0.01 wt% to about 1 wt%, more preferably from about 0.01 wt% to about 0.5 wt% relative to the total weight of the bleach composition.

### Nonionic Surfactant Additive

Nonionic surfactant additive for embodiments of the bleach composition includes but is not limited to polyethoxylated and polypropoxylated, alkylphenols, α-diols or alcohols having a fatty chain containing, for example, 8 to 18 carbon atoms, with the number of ethylene oxide or propylene oxide groups to range in particular from 2 to 50. Surfactant copolymers of ethylene oxide and of propylene oxide with fatty alcohols, fatty amides, fatty acid esters also serve as a nonionic surfactant additive for embodiments of the bleach composition. These are condensates of ethylene oxide and of propylene oxide with fatty alcohols; polyethoxylated fatty amides preferably having from 2 to 30 mol of ethylene oxide, polyglycerolated fatty amides containing on average 1 to 5, and in particular 1.5 to 4, glycerol groups. Still further nonionic surfactant additives for the Bleach Composition include oxyethylenated fatty acid esters of sorbitan having from 2 to 30 mol of ethylene oxide; fatty acid esters of sucrose, fatty acid esters of polyethylene glycol; alkylpolyglycosides; N-alkylglucamine derivatives.

### Amphoteric Surfactant Additive

The amphoteric (or zwitterionic) surfactant additive for embodiments of the bleach composition includes but is not limited to aliphatic secondary or tertiary amine derivatives in which the aliphatic radical is a linear or branched chain containing 8 to 18 carbon atoms and containing at least one water-solubilizing anionic group, for example carboxylate, sulphonate, sulphate, phosphate or phosphonate. Exemplary amphoteric surfactant additives include (Cs-C₂₀)alkylbetaines, sulphobetaines, (C₈-C₂₀) alkylamido(C₁- C₆)alkylbetaines or (C₈-C₂₀)alkylamido(C₁-C₆)alkylsulphobetaines.

Exemplary amine derivatives also include the products sold by the company Rhodia Chimie under the trade name Miranol^{®} and are described chemically as "disodium cocoamphodiacetate", "disodium lauroamphodiacetate", "disodium caprylamphodiacetate", "disodium capryloamphodiacetate", "disodium cocoamphodipropionate", "disodium lauroamphodipropionate", "disodium caprylamphodipropionate", "disodium capryloamphodiprbpionate", "lauroamphodipropionic acid" and "cocoamphodipropionic acid".

### Cationic Surfactant Additive

The cationic surfactant additive for embodiments of the bleach composition includes but is not limited to the cationic surfactant of the hair conditioner described above. Exemplary cationic surfactants include salts of optionally polyoxyalkylenated primary, secondary or tertiary fatty amines; quaternary ammonium salts such as tetraalkylammonium, alkylamidoalkyltrialkylammonium, trialkylbenzylammonium, trialkylhydroxyalkylammonium or alkylpyridinium chlorides or bromides; imidazoline derivatives; and amine oxides of cationic nature.

### Amino Acid Additive

The optional amino acid additive for embodiments of the bleach composition may include one or more natural, non-natural and/or synthetic amino acids. The amino acid additive may be included in embodiments of the bleach composition especially when protection from undesirable free radicals may be produced during the oxidation process. Embodiments of the bleach composition may include concentrations of one or more amino acids of from about 0.01 wt% to about 5 wt%, preferably about 0.01 wt% to about 2 wt%, more preferably about 0.01 wt% to about 1 wt%, especially more preferably about 0.01 wt% to about 0.1 wt% relative to the total weight of the bleach compoisiton. Preferred amino acids for inclusion in embodiments of the bleach composition are hydrophobic amino acids.

Natural amino acids include Isoleucine (Ile), Leucine (Leu), Valine (Val), Alanine (Ala), Phenyl alanine (Phe), Tryptophan (Trp), Methionine (Met), Tyrosine (Tyr), Glutamine (Gln), Glutamate/Glutamic acid (Glu), Glycine (Gly), Histidine (His), Lysine (Lys), Proline (Pro), Serine (Ser), Tyrosine (Tyr), Tryptophan (Trp), Methionine (Met), Aspartic Acid (Asp), Asparagine (Asn). Of these, those that are sensitive to base degradation and to oxidation are disfavored. Also amino acids disfavored in cosmetic preparations such as phenyl alanine are also disfavored. Non-natural amino acids having a hydrophobic character are also included. These non-natural amino acids include but are not limited to 2, 3, 4, 5 or 6-amino C3-C6 alkanoic acid. Examples include 3-amino propionic acid, 2-amino butyric acid, 3-amino butyric acid, 4-amino butyric acid, 2-amino pentanoic acid, 3-amino pentanoic acid, 4-amino pentanoic acid, 5-amino pentanoic acid, 2-amino hexanoic acid, 3-amino hexanoic acid, 4-amino hexanoic acid, 5-amino hexanoic acid, 6-amino hexanoic acid. Preferred amino acids for use with embodiments of the bleach powder of the invention comprise at least one of glycine, serine, glutamate, asparagine, lysine, arginine, histidine threonine, isoleucine, leucine, valine, alanine, homoalanine or any combination thereof. More preferred are arginine, asparagine, lysine and glutamate.

### Carboxylic Acid Additive

The optional additive for embodiments of the bleach composition may also optionally include a di or tri carboxylic acid. Exemplary carboxylic acids include but are not limited to a C2-C10 di or tricarboxylic acid with optional hydroxyl group particularly preferably selected from succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso-tartaric acid, racemic acid, alpha- ketoglutaric acid, beta-ketoglutaric acid, oxaloacetic acid, tartronic acid, hydroxyglutaric acid and/or at least one salt of these acids and mixtures of these compounds, wherein the at least one dicarboxylic acid having 2 to 10 carbon atoms is selected from succinic acid, malic acid and adipic acid and salts thereof.

Salts of dicarboxylic acids having 2 to 10 carbon atoms that are preferred as contemplated herein are selected from the mono salts and di salts of the anions of succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso- tartaric acid, racemic acid, alpha-ketoglutaric acid, beta- ketoglutaric acid, oxaloacetic acid with ammonium ions, alkali metal ions, alkaline earth metal ions and the ions of basic amino acids, such as arginine, lysine and histidine, in particular with lithium, sodium, potassium, magnesium and calcium ions, and mixtures of these salts.

Incorporation of a solid carboxylic acid and preferably solid carboxylic acid salt into embodiments of the bleach composition can provide a protection function to the keratin protein against the oxidative nature of the aqueous or aqueous organic medium with dissolved bleach powder.

Embodiments of the bleach composition may include a di or tri carboxylic acid at a concentration of from about 0.1 wt% to about 5 wt%, preferably from about 0.1 wt% to about 2 wt%, more preferably from about 0.1 wt% to about 1 wt% relative to the total weight of the bleach composition.

### Disintegrant Additive

The optional disintegrant additive for embodiments of the bleach composition enable facile dissolution and/or dispersion of the solid and/or paste form of embodiments of the bleach composition into aqueous medium and/or developer. Exemplary disintegrants include croscarmellose, crosslinked cellulose, crosspovidone, crosslinked PVP, starch, hydrated starch, starch glycolate, crosslinked alginic acid and similar swelling and/or wicking compounds

Embodiments of the bleach composition may include a disintegrant at a concentration of from about 0.01 wt% to about 1 wt%, preferably from about 0.01 wt% to about 0.1 wt% relative to the total weight of the bleach composition.

### Solvent Additive

An optional solvent additive for embodiments of the bleach composition may be included in a minor concentration such as from about 0.1 wt% to about 5 wt%, preferably up to about 3 wt % more preferably from about 0.1 wt% to about 1 wt% relative to the total weight of the embodiments of the bleach composition. Examples include water and aqueous mixtures composed of water and one or more cosmetically acceptable organic solvents, such as an alcohol including ethanol, isopropanol, benzyl alcohol, phenylethyl alcohol or cetyl alcohol; polyols, for instance propylene glycol and glycerol; glycol ethers, for instance ethylene glycol monomethyl ether, monoethyl ether and monobutyl ether, and also glycol alkyl ethers, for instance diethylene glycol monoethyl ether or monobutyl ether. The solvent is optionally present at least in part to enable substantial homogenization of solid components when combined together to form a solid particulate or powder or to form a paste.

### Filler Additive

One or more optional filler additives for embodiments of the bleach composition include inorganic salts, clays, colloids and similar inert, insoluble substances. The optional filler additive overlaps to an extent with rheology modifiers and thickeners described above.

A particulate filler may but not necessarily be a particulate with a particle size of about 0.1 to about 250 microns. Included are inorganic, inert, insoluble substances, inorganic salts, colloids, carbohydrates, soap salts and alkyl sulfate salts. Exemplary inorganic fillers include but are not limited to silica, hydrated silica, attapulgite, bentonite, calcium oxide, chalk, diamond powder, diatomaceous earth, fuller's earth hectorite, kaolin, mica, magnesium oxide, magnesium peroxide, montmorillonite, pumice, talc, tin oxide, zeolite, zinc oxide, and the like.

Examples of suitable hydrophilic colloids include hydroxyethylcellulose, locust bean gum, maltodextrin, methylcellulose, agar, dextran, dextran sulfate, gelatin, pectin, potassium alginate, sodium carboxymethylchitin, xanthan gum, and the like.

Examples of suitable carbohydrates include sugars such as glucose, sucrose, maltose, xylose, trehelose, and derivatives thereof, in particular sugar esters of long chain, C14_30 fatty acids, as well as dextrins, cellulosics, and derivatives thereof.

Examples of soaps and alkyl sulfate particles that may act as particulate fillers include the aluminum, sodium, and potassium salts of fatty acids such as aluminum distearate, aluminum isostearate, aluminum myristate, calcium behenate, calcium stearate, calcium behenate, magnesium stearate, magnesium tallowate, potassium palmitate, potas- sium stearate, potassium oleate, sodium stearate, sodium oleate, sodium myristate, sodium palmitate, and the like. Suitable alkyl sulfates include sodium lauryl sulfate, sodium cetyl sulfate, sodium myristyl sulfate, sodium octyl sulfate, and the like.

At least some of these examples of filler additives have additional functions as additives described in other additive sections herein. As a filler additive, the concentration of the substance may be from about 0.1 wt% to at much as about 10 wt%, preferably from about 0.1 wt% to about 2 wt%, more preferably from about 0.1 wt% to about 1 wt% relative to the total weight of the bleach composition.

### Silicone Additive

Optional silicone additives for Embodiments of the bleach composition include linear, branched, cyclic, cage and net polysilicones, oligosilicones and derivatives. Examples include but are not limited to silicone oils such as decamethylcyclopentasiloxane, dodecamethylcyclohexasiloxane, dimethylpolysiloxane and methylphenylpolysiloxane, as well as hexamethyldisiloxane, octamethyltrisiloxane and decamethyltetrasiloxane. Further examples of the silicones include dimethicones, dimethiconols, cyclic silicones, and modified silicones with various functional groups such as amino groups, quaternary ammonium salt groups, and amine silicones such as Amodimethicone . Such silicones may be soluble or insoluble in the aqueous (or aqueous-organic) medium. In the case of insoluble liquid silicones, the polymer can be in an emulsified form with droplet size of about 10 nm to about 30 micrometers.

The optional silicone additive may function as a hair conditioner as well as a coating agent for microparticulates forming the particulate or powder bleach composition embodiments. The optional silicone additive may also be separately added to the hair following bleaching and rinsing. In this option, the silicone additive may be combined with one or more of the fatty substances and hair conditioners described above to be a free standing conditioner composition to be used on the hair post bleaching.

### Bleach Composition Examples

Examples of Bleach Compositions include:
A. A solid powder or granular combination of the inorganic peroxo oxidizer, the chelate mixture and alkalizer alone with the proportions given above.
B. A solid powder or granular combination of the oxidizers sodium and/or potassium persulfate, the chelate mixture and sodium silicate and ammonium hydroxide alone with the proportions given above.
C. A solid powder or granular combination of the oxidizers sodium and/or potassium persulfate, the chelate mixture, sodium silicate and/or sodium metasilicate and ammonium hydroxide and/or sodium hydroxide alone with the proportions given above.
D. A solid powder or granular combination of the oxidizers sodium and/or potassium persulfate and/or potassium percarbonate, the chelate mixture, sodium silicate and/or sodium metasilicate and/or ammonium carbonate and/or sodium carbonate alone with the proportions given above.
E. A solid powder or granular combination of the oxidizers sodium and/or potassium persulfate and/or potassium percarbonate, the chelate mixture, sodium silicate and/or sodium metasilicate and/or ammonium carbonate alone with the proportions given above.
F. A solid powder or granular combination of the oxidizers sodium and/or potassium persulfate and/or potassium percarbonate and/or sodium perborate, the chelate mixture, sodium silicate and/or sodium metasilicate and/or sodium hydrated silicate and/or ammonium carbonate alone with the proportions given above.
G. A solid powder or granular combination of the oxidizers sodium and/or potassium persulfate and/or potassium percarbonate, the chelate mixture, sodium silicate and/or sodium metasilicate and/or sodium hydrated silicate and/or ammonium and/or potassium phosphate and/or ammonium carbonate alone with the proportions given above.
H. A solid powder or granular combination of the oxidizers sodium and potassium persulfate and potassium percarbonate and/or perbicarbonate and/or sodium perborate, the chelate mixture, sodium silicate and/or ammonium hydroxide, ammonium carbonate, sodium metasilicate alone with the proportions given above.
I. A solid powder or granular combination of the oxidizers sodium and potassium persulfate, potassium percarbonate and perbicarbonate, optional sodium hypochlorite and chlorite, the chelate mixture and sodium silicate, ammonium carbonate, sodium metasilicate and carbonate and phosphate buffer alone with the proportions given above.
J. A solid powder or granular combination of the oxidizers sodium and potassium persulfate, potassium percarbonate and perbicarbonate, the chelate mixture and sodium silicate, ammonium carbonate, sodium metasilicate, a carbonate and phosphate buffer, a detergent of sodium or potassium lauryl sulfate, a fatty alcohol of ceteryl alcohol and paraffin oil alone with the proportions given above.
K. A solid powder or granular combination of the oxidizers sodium and potassium persulfate, potassium percarbonate and perbicarbonate, the chelate mixture and sodium silicate, ammonium carbonate, sodium metasilicate, a carbonate and/or phosphate buffer, a detergent of sodium or potassium lauryl sulfate, a fatty alcohol such as cetearyl alcohol and xanthan gum alone with the proportions given above.
L. A solid powder or granular combination of the oxidizers sodium and potassium persulfate, potassium percarbonate and perbicarbonate, the chelate mixture and sodium silicate, ammonium carbonate, sodium metasilicate, a carbonate and/or phosphate buffer, a detergent of sodium or potassium lauryl sulfate and/or sodium or potassium laureth sulfate, a fatty alcohol such as stearyl alcohol and/or cetearyl alcohol, an optional fatty amine such as stearyl amine and/or stearyl diethyl amine alone with the proportions given above.
M. Addition of a thickener such as one or more of guar gum, xanthan gum, agar as a single addition to any of the above Bleach Composition examples.
N. Addition of a hydrocarbon such as paraffin oil, squalane or farnesane as a single addtin to any of the above Bleach Composition examples.
O. Combination of any of the above solid powder or granular Bleach Composition examples with from 20 wt.% to 50 wt.% water to form an aqueous bleaching formulation.
P. Combination of any of the above solid powder or granular Bleach Composition examples with an equal weight of 12 % aqueous hydrogen peroxide and adjustment of the pH to be between 9 and 10.
Q. Combination with any of the above solid powder or granular Bleach Composition examples with double the weight of 12 % aqueous hydrogen peroxide and adjustment of the pH to be between 9 and 10.

### Bleach Composition Method of Preparation A

Preparation of a solid particulate or powder form of embodiments of the bleach composition or a paste form of embodiments of the bleach composition begins with combining the three essential components, the oxidizing agent, the chelate mixture and the alkalizing agent. Because as individual starting materials, these three components are mixtures of inorganic and/or organic peroxo compounds, mixtures of the three chelates, EDDS, MGDA and EDTA and mixtures of the individual ingredients for the alkalizing agent, the ingredients for these starting materials are premixed according to preferences for the individual inorganic and/or organic peroxo compound ingredients, the individual chelate ingredients and the individual alkalizing ingredients. Usually, the individual ingredients for the peroxo compounds include sodium and/or potassium persulfate, sodium and/or potassium percarbonate as well as either or both of these inorganic peroxo compounds with either or both of optional phthaloyl peroxide and t-butyl peroxybenzoate. Preferably the oxidizing agent is a mixture of the two inorganic peroxo compounds.

Next the mixture of EDDS, MGDA and EDTA is made by combining these three chelates according to any of the weight part ranges and the total weight range of the chelate mixture given above.

Finally, the alkalizing agent is formed as a mixture of sodium silicate and ammonium hydroxide, or sodium silicate and ammonium carbonate at a molar ratio of 1: 1 to 2:1.

A surfactant such as a sodium or potassium C14 -C24 alkanoate salt or alkan-1-sulfate salt or alkan-1-sulfonate salt; a guar gum, a xanthan gum or locust bean gum as a thickener and petroleum jelly, paraffin oil or mineral oil as a hydrocarbon can be added to perform their recognized functions. A phosphate buffer, a fatty alcohol and an oil can be added to protect scalp and skin and lessen the potency of the oxidizer.

An example of the mixture of oxidizing agent, chelate mixture and alkalizing agent is formed by combining 8 weight parts oxidizing agent which is a combination of sodium persulfate and potassium percarbonate at a 1:2 molar ratio, 1 part chelate mixture and 1 part alkalizing agent of sodium silicate and ammonium carbonate at a 1:1 molar ratio to form an unprocessed, heterogeneous combination of components constituting an embodiment of the bleach composition.

The unprocessed combination can be placed in a closed ball mill and ground with 5 millimeter balls to an average particle size of about 5 millimeter of heterogeneous particles. The contents of the ball mill are removed and the 5 millimeter balls are separated from the hetergeneous particles. The heterogeneous particles are introduced into a mixing container with a removable agitator and combined with several additives to form an additive-bleach combination. The additive-bleach combination is made with 100 weight parts of the heterogeneous particles, 2 weight parts sodium stearate, 2 weight parts cetearyl alcohol, 0.3 weight parts xanthan gum and 0.3 weight parts hectorite or silica. The additive-bleach combination is mixed by high speed agitator to form a substantially homogeneous mixture of the additive-bleach combination. The agitator is removed and mill balls of 100 microns size are introduced into the mixing container and the additive-bleach combination milled to an average 250 micron size. The milling should proceed for at least 5 minutes. At about half way through milling, an aliquot of the milled bleach composition embodiment is taken and the particles tested for homogeneity of oxidizing agent, chelate mixture and alkalizing agent from particle to particle. If the particles do not show substantial homogeneity from particle to particle, about 0.01 to 0.05 wt percent aqueous isopropanol relative to the total weight of the milled mixture wherein the aqueous isopropanol is about 95-99 vol % isopropanol, about 1 to 5 vol % water is added and the particles milled for at least another 5 minutes or until a sampled aliquot shows that the particles test successfully for homogeneity. The milled product is a solid particulate/powder embodiment of the bleach composition of the invention.

To form a paste of the solid particulate/powder embodiment of the bleach composition of the invention, from about 2 wt% to about 10 wt% of mineral hydrocarbon such as mineral oil or liquid paraffin relative to the total weight of the particulate powder embodiment is added to the particulate/powder embodiment to form a complex mixture; or a combination of the same amount of mineral hydrocarbon in mixture with a combination of a C16-C30 fatty alcohol and a C16-C30 fatty acid such as ceryl alcohol and arachidic acid at about 0.01 wt% to about 0.05 wt% relative to the total weight of the particulate/ powder embodiment is added to the particulate/powder embodiment to form a complex mixture. The complex mixture is stirred at high speed until a paste with substantially uniformly distributed particulate/powder embodiment is produced.

### Bleach Composition Method of Preparation B

Preparation of a solid particulate or powder form of embodiments of the bleach composition or a paste form of embodiments of the bleach composition begins with combining together the three essential components, the oxidizing agent, the chelate mixture and the alkalizing agent. Because all three components are mixtures of the individual starting material compounds, the individual inorganic and/or organic peroxo compounds, the individual three chelates, EDDS, MGDA and EDTA and the individual ingredients of the alkalizing agent are individually premixed according to the compound and amount preferences to prepare individually the three components of the bleach composition. Usually, the individual ingredients for the peroxo compounds include sodium and/or potassium persulfate, sodium and/or potassium percarbonate as well as either or both of these inorganic peroxo compounds with either or both of optional phthaloyl peroxide, t-butyl peroxybenzoate. Preferably the oxidizing agent is a mixture of the two inorganic peroxo compounds. The individual compounds of the alkalizing agent typically include sodium silicate with ammonium hydroxide and/or ammonium carbonate.

The multiple individual starting ingredients for these three components are individually pre-milled, preferably by the ingredient vendor, to a particle size of 50 microns or smaller. The pre-milled sizing of the individual ingredients for the oxidizing agent, the chelate mixture and the alkalizing agent facilitate ready formulation of a substantially homogeneous bleach composition.

Appropriate weight portions of the primary components of the bleach composition, the peroxo compound or compounds, each of the chelates of the chelate mixture and each of the one or more alkali compounds of the alkalizing agent are provided and transported to the mixing container of a high-speed solids mixing device with milling blades.

A device lid is closed onto the container of the mixing device and the milling blades of the device are actuated to comminute to mixture of the primary components for a period of not less than 5 minutes to form a fine particulate powder of the primary components. Optional additives are measured to prepare their appropriate weight portions and each individual additive, if any, is pre-milled or ground to a particle size of no larger than 50 microns. The ground additive is combined sequentially with the particulate powder of the primary components for at least 2 minutes of high-speed mill stirring for each additional additive. If the additive is an oil, is sticky, and/or is agglomerated, the additive is premixed with a minimal amount of organic solvent and/or water to form a dispersion and/or solution of the additive. The dispersion and/or solution of the additive is added following combination of all other solid, pre-milled additives and the particulate powder of the primary components. A substantially homogeneous particulate powder of the bleach composition optionally with one or more additives is prepared by this method.

If desired, the substantially homogeneous particulate powder of the bleach composition can be reformulated as granules by mixing the powder with a solid binder and disintegrant at appropriate portions. Following mixing of the particulate powder of the bleach composition and the binder with disintegrant to form a binder mixture, small 10 to 50 mg portions of the binder mixture can be compressed to form granules of embodiments of the bleach composition. The granules enable ready measurement of this embodiment of the bleach composition.

### Preparation of a Bleaching Formulation

Embodiments of the bleach composition prepared as described above may be transformed into a bleaching formulation by combining the solid particulate, powder or paste embodiment of the bleach composition described above with sufficient water, preferably deionized water, or with peroxide developer composition, to produce a semi-fluid bleaching formulation. The addition of water or peroxide developer composition dissolves the peroxo oxidizer and alkalizer to produce the basic pH semi-fluid bleaching formulation that will deliver at least peroxo radicals as oxidizing moieties. The bleaching formulation formed with water or with peroxide developer is prepared by combining the bleach composition with water or the peroxide developer at a wt to wt ratio of 1:1.5 or 1:2 or 1:3.

The peroxide developer composition used to form the above described alternative of the bleaching formulation includes at least hydrogen peroxide and may optionally comprise a fatty alcohol such as cetyl, stearyl, or cetearyl alcohol, an anionic surfactant such as sodium or potassium stearate, cetearyl sulfate and/or laureth sulfate; a pH buffer such as a disodium phosphate/phosphoric acid buffer, a disodium phosphate/citric acid buffer, a boric acid/sodium tetraborate buffer or a borate buffer, each of which is adjusted to provide an acidic pH; and an acid such as etidronic acid and/or salicylic acid and/or malic acid.

Exemplary embodiments of the bleach composition may comprise at least the oxidizer, chelator mixture and alkalizing agent described above. The oxidizer may be a peroxy salt, the chelator may be the mixture of the three chelators EDTA, EDDS and MGDA and the alkalizer may be an inorganic compound forming a basic pH in aqueous medium. The preferred peroxy salt may be a persulfate salt such as sodium, potassium and/or ammonium persulfate. The alkalizing agent may be sodium silicate and magnesium or ammonium carbonate/hydroxide.

Additional, preferred additives include an anionic surfactant, a fatty alcohol, a hydrocarbon, a buffer and a thickener. Specific ingredients for these classes of additives include an anionic surfactant of sodium or potassium stearate, cetearyl sulfate and/or laureth sulfate; a hydrocarbon such as mineral oil or liquid paraffin; a thickener such as xanthan gum, algin, locust bean gum, guar gum and a fatty alcohol such as cetyl, stearyl, or cetearyl alcohol.

### The Bleaching Method

The method for bleaching the hair of a person can be accomplished by preparing an aqueous bleaching formulation from an embodiment of the solid particulate, powder or paste bleach composition and water, preferably deionized water as described above. Alternatively, the particulate, powder or paste embodiment of the bleach composition may be combined with the peroxide developer as described above. The bleaching formulation prepared by either method has a pH of from about 9 to 11 and an oxidation capacity delivering an oxidizing equivalent of from about 3 wt% to about 12 wt%, preferably from about 3 wt% to about 6 wt % of hydrogen peroxide. The bleaching formulation prepared from a wt% ratio of bleach composition to water or a wt% ratio of bleach composition to peroxide developer follows the wt to wt ratio given above (e.g., wt to wt ratio of 1:1.5 or 1:2 or 1:3). Preferably the wt to wt ratio is about 1: 1.5 wherein the bleach composition is measured as the solid particulate or powder form. The viscosity of the bleaching formulation should exhibit a viscosity similar to that of ketchup or mustard dressing.

The hair on the head of a person is first rinsed with water and dried to dampness. The whole head of hair is sectioned by dividing the hair into two halves and 3 or 4 separate swatches from each half. The swatches that are not to be immediately treated can preferably be isolated by covering them with foil. The isolated hair swatch to be bleached is painted, brushed or otherwise treated with a first embodiment of the bleaching formulation wherein the oxidizing equivalent of this first embodiment is equivalent to about 3 wt% to about 6 wt% of aqueous hydrogen peroxide. The painting or brushing should begin about an inch from the hair roots and slowly continued to the tips. The swatch should be turned upside down by lifting and the underside of the swatch painted in the same fashion. The bleaching formulation should be worked into the hair swatch by gently massaging it into the interior of the swatch so that all hair strands of the swatch are thoroughly coated. The time for applying the bleaching formulation to the swatch and for massaging should be no longer than about 2 to 3 minutes. The additional swatches can be treated in this same fashion.

Once all swatches are treated on both sides with the bleaching formulation, the roots of the swatches are preferably treated with a second embodiment of the bleaching formulation having a higher oxidating equivalent of oxidizer than is present for the first embodiment of the bleaching formulation. This second equivalent typically has an oxidizing equivalent of about 6 wt% to about 9 wt% aqueous hydrogen peroxide.

The total time for bleaching relative to the last application should be approximately at least about 15 minutes. The decolorization of the hair swatches at the roots and at the remainder of the hair strands is checked to determine uniform decolorization. If there are sections of swatches that are darker than others, these sections are retreated with the first embodiment of the bleaching formulation to assure substantially uniform decolorization.

The entire head of hair is rinsed with water to remove residual bleaching formulation. Although shampooing is not necessary, it can be performed if the hair feels sticky or slick. This feeling usually indicate incomplete removal of the bleaching formulation.

### Oxidative Dye Coloring Following Bleaching

Embodiments of the bleaching formulation may be employed as described above to pretreat hair to provide lightening and/or decolorization in preparation for oxidatively dyeing the hair with an aqueous oxidative dye composition of a basic oxidizer, at least one precursor dye and at least one coupler dye in an aqueous chassis. The basic oxidizer and chassis for the oxidative coloring procedure are described below. The at least one oxidation precursor dye and the at least one coupler dye are introduced into an aqueous medium for the oxidative dye composition at amounts of each of from about 0.0001 to about 10.0% by weight, preferably from about 0.001 to about 8% by weight relative to the total weight of the aqueous oxidative dye composition.

The precursor and coupler ingredients are generally used in approximately equimolar amounts to one another. To hasten the oxidative reaction, a minor excess of the coupler may sometimes be employed. In such situations, the weight percentage sum of all couplers present may be slightly larger than the weight percentage sum of all precursors present.

Typical precursors for the aqueous oxidative dye composition utilize an oxidatively susceptible aromatic compound substituted by at least one amine group. Exemplary precursors include one or more of, and/or any combination of 2-methoxymethyl-p-phenylene diamine (MBB), p-aminophenol (PAP), hydroxyethyl-p-phenylenediamine sulphate, hydroxypropyl bis(n-hydroxyethyl-p-phenylenediamine), 2-chloro-p-phenylenediamine, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diamino dihydroxypyrazolo pyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and addition salts, solvates and/or solvates of salts thereof.

Additional precursors include one or more of, and/or any combination of p-phenylenediamine, p-toluenylamine, 2-(2-hydroxyethyl)-p-phenylenediamine, 2-(1,2-dihydroxyethyl)-p-phenylenediamine, N,N-bis-(2-hydroxyethyl)-p-phenylenediamine, N-(4-amino-3-methylphenyl)-N-[3-(1H-imidazol-1-yl)propyl]amine, N,N'-bis-(2-hydroxyethyl)-N,N'-bis-(4-aminophenyl)-1,3-diaminopropan-2-ol, bis-(2-hydroxy-5-aminophenyl)methane, 1,3-bis-(2,5-diaminophenoxy)propan-2-ol, N,N'-bis-(4-aminophenyl)-1,4-diazacycloheptane, 1,10-bis-(2,5-diaminophenyl)-1,4,7,10-tetraoxadecane, p-aminophenol, 4-amino-3-methylphenol, 4-amino-2-aminomethylphenol, 4-amino-2-(1,2-dihydroxyethyl)phenol, 4-amino-2-(diethylaminomethyl)phenol, 4,5-diamino-1-(2-hydroxyethyl)pyrazole, 2,4,5,6-tetraamino pyrimidine, 4-hydroxy-2,5,6-triaminopyrimidine, 2-hydroxy-4,5,6-triaminopyrimidine, and the addition salts, solvates and/or solvates of salts thereof.

Typical couplers for the aqueous oxidative dye composition utilize an electrophilic acceptor aromatic compound substituted by at least one hydroxyl group. However, cosmetically disfavored couplers such as resorcinol, alkyl resorcinol, halo resorcinol or alkyl halo resorcinol are excluded for use herein.

Exemplary couplers include one or more of and/or any combination of hydroxyethyl-3,4-methylenedioxyaniline (Aminol), 2,4-diaminophenoxyethanol (DPE),; m-aminophenol (MAP), 4-amino-2-hydroxy toluene (AHT), ethyl ortho-amino phenol (EOAP), , 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 2-amino-5-ethyl phenol, 6-hydroxybenzomorpholine, 6-amino-m-cresol, 6-amino-o-cresol, 2-amino-4-hydroxyethylamino-anisole, 1,3-bis-(2,4-diaminophenoxy)propane, 2,6-dihydroxyethylaminotoluene, 2,4-diamino-1,5-di(2-hydroxyethoxy)benzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindoline, phenyl methyl pyrazolone, hydroquinone, 4-formyl-1-methylquinolinium-p-toluenesulfonate; and the addition salts, the solvates and/or solvates of salts thereof.

Additional couplers include 3-aminophenol, 5-amino-2-methylphenol, N-cyclopentyl-3-aminophenol, 3-amino-2-chloro-6-methylphenol, 2-hydroxy-4-aminophenoxyethanol, 2,6-dimethyl-3-aminophenol, 3-trifluoroacetylamino-2-chloro-6-methylphenol, 5-amino-4-chloro-2-methylphenol, 5-amino-4-methoxy-2-methylphenyl, 5-(2-hydroxyethyl)amino-2-methylphenol, 3-(diethylamino)phenol, N-cyclopentyl-3-aminophenyl, 1,3-dihydroxy-5-(methylamino)benzene, 3-ethylamino-4-methylphenol, 2,4-dichloro-3-aminophenol, 2-(2,4-diaminophenoxy)ethanol, 1,3-bis(2,4-diaminophenoxy)propane, 1-methoxy-2-amino-4-(2-hydroxyethylamino)benzene, 1,3-bis(2,4-diaminophenyl)propane, 2,6-bis(2'-hydroxyethylamino)-1-methylbenzene, 2-({3-[(2-hydroxyethyl)amino]-4-methoxy-5-methylphenyl}amino)ethanol, 2-({3-[(2-hydroxyethyl)amino]-2-methoxy-5-methylphenyl}amino)ethanol, 2-({3-[(2-hydroxyethyl)amino]-4, 5-dimethylphenyl}amino)ethanol, 2-[3-morpholin-4-ylphenyl)amino]ethanol, 3-amino-4-(2-methoxyethoxy)-5-methylphenylamine, 1-amino-3-bis-(2-hydroxyethyl)aminobenzene, pyrogallol, 2-amino-3-hydroxypyridine, 2-amino-5-chloro-3-hydroxypyridine, 3-amino-2-methylamino-6-methoxypyridine, 2,6-dihydroxy-3,4-dimethylpyridine, 2,6-dihydroxy-4-methylpyridine, 2,3-diamino-6-methoxypyridine, 3,5-diamino-2, 6-dimethoxypyridine, 2, 6-dihydroxy-3,4-dimethylpyridine, 2-(2-methoxyethyl)amino-3-amino-6-methoxypyridine, 2-(4'-methoxyphenyl)amino-3-aminopyridine, 1-naphthol, 2-methyl-1-naphthol, 2-hydroxymethyl-1-naphthol, 2-hydroxyethyl-1-naphthol, , 1,5-dihydroxynaphthalene, 1, 6-dihydroxynaphthalene, 1, 7-dihydroxynaphthalene, 1,8-dihydroxynaphthalene, 2,7-dihydroxynaphthalene, 2,3-dihydroxynaphthalene, 4-hydroxyindole, 6-hydroxyindole, 7-hydroxyindole, 4-hydroxyindoline, 6-hydroxyindoline, 7-hydroxyindoline, 4-6-diaminopyrimidine, 4-amino-2,6-dihydroxypyrimidine, 2,4-diamino-6-hydroxypyrimidine, 2,4,6-trihydroxypyrimidine, 2-amino-4-methylpyrimidine, 2-amino-4-hydroxy-6-methylpyrimidine and 4,6-dihydroxy-2-methylpyrimidine or mixtures of these compounds or the physiologically acceptable salts thereof.

The aqueous oxidative dye composition of one or more of the above described precursor(s) and coupler(s) is completed by addition of the alkalizing composition, the chassis and sufficient hydrogen peroxide in acidic aqueous medium (preferably with etidronic and salicylic acids) to form about 3 wt% to about 12 wt%, preferably about 3 wt% to about 8 wt%, more preferably about 3 wt% to about 6 wt percent of hydrogen peroxide. The acidic aqueous hydrogen peroxide becomes a basic aqueous medium through addition of a concentration of alkalizing composition calculated to raise the acidic pH of the acidic aqueous hydrogen peroxide to a pH of from 8 to 11, preferably 8 to 9. The alkalizing composition comprises one or more of hydroxide, metasilicate, carbonate with sodium, potassium and/or ammonium cations preferably a combination of ammonium hydroxide and sodium or potassium carbonate. The chassis of dicetyl phosphate, ceteth-10 phosphate, cetearyl alcohol, glycerin and propylene glycol, ascorbic acid, and xanthan gum rounds out the ingredients mixture for the aqueous oxidative dye composition.

### The Oxidative Dyeing Process

The bleached head of hair prepared as described above through use of the bleaching formulation is prepared for oxidatively dyeing by separating the hair into swatches as described above for the bleaching process. The swatches other than the one to be oxidatively dyed are covered with foil.

A dye composition as described above and containing at least one precursor, at least one coupler, the oxidizer component, alkalizer and chassis as described above is prepared with the concentrations described above.

A portion of the dye composition is applied to the both sides of the hair swatch by painting from about half an inch above the roots to the hair strand tips. A second portion of the dye composition prepared with about 10 to 20 wt% higher concentrations of precursor and coupler is applied to the root portions of the swatch.

The remaining swatches are treated in the same manner and the composite swatches are visually inspected to assure that the dyeing has achieved the desired result. The dye composition is allowed to remain on the hair for a period of time of about 20 to 30 minutes after the last swatch has been treated.

At the end of the oxidative dyeing process, all components of the aqueous oxidative dye composition are rinsed from the hair using water or a surfactant-containing cleansing agent. A commercially available shampoo can be used in particular as cleansing agent, although use of a shampoo can be eliminated if the aqueous oxidative dye composition includes a surfactant such as an anionic or cationic surfactant. The whole head of hair is then rinsed with copious quantities of water as described above. In this fashion a new hair color for a whole head of hair can be produced.

### EXPERIMENTAL SECTION

The experimental examples present the protocols and results for the foaming study, the lift study, the damage to lift study and the temperature control study. The foaming study used a hydrogen peroxide developer styled as Welloxon. The selected metal ions as their salts, the selected chelators and additional Bleach Composition ingredients were added to the Welloxon material to produce the foaming compositions studied.

The lift and damage studies were conducted with hair swatches. The Welloxon developer contained from 12 wt% to 24 wt% aqueous solution of 50% hydrogen peroxide so as to deliver 6 wt% to 12 wt% hydrogen peroxide as well as sodium ceteryl sulfate, phosphoric acid/disodium phosphate buffer, etidronic and salicylic acids and bluing agent. The pH of the Welloxon material was 2.3 to 2.9.

### Preparation of Bleach/Metal Ion/Chelator Test Mixtures

The bleach formulation followed the description of the above described Bleach Composition except for chelator(s). The bleach formulation for conducting the experimental tests included potassium and ammonium persulfate as oxidizers, magnesium carbonate and sodium silicate as alkalizer, sodium stearate as an anionic surfactant, silica, xanthan gum thickener, liquid paraffin as a hydrocarbon oil, malic acid as a keratin fiber protectant, and a bluing agent or laundry bluing such as ultramarines. The relative weight percentages of these ingredients follow the disclosures given above. When the bleach formulation is mixed with the above described Welloxon developer at a wt-wt ratio of 1:1.5 the pH of the resulting pro-bleaching formulation is 8.5-10.

### Foaming Study Protocol with Chelators

A pre-foam mixture is prepared by combining a 20 g portion of the above described bleach formulation with 40 ml of Welloxon^{®} developer 9% (pro-bleaching formulation). To this pre-foam mixture was added the chelator as described below to form the chelator-bleaching formulation. The chelator as 1 ml of 1% copper(II) sulfate or 1% iron (III) sulfate or a 1 ml mixture of 1% copper (II) sulfate and 1% iron (III) sulfate is then added. The resulting foam mixture was stirred to prepare a uniform dispersion/solution of the foamable liquid.

The method for creating foam was practiced by pouring the foamable liquid into a 250 ml beaker and placing into an oven at 30°C with air circulation to cause the hydrogen peroxide redox reaction catalyzed by copper and/or iron to produce a solid foam. After foaming and drying for approximately 15 minutes , the beaker with solid foam was removed from the drying cupboard and the foam height measured.

To study the foam capacity of EDTA, EDDS and MGDA, each additional chelator at 1 wt% or 2 wt% was added to the above described pre-foam mixture, the pre-foam mixture combined with copper sulfate or iron sulfate or a mixture thereof as described above and the beaker with the foamable liquid was processed as described above to provide a beaker with solid foam.

The beaker foams are produced with:
a) EDTA foam a with 1% copper sulfate
b) EDTA foam b with 1% iron sulfate
c) EDTA foam c with a combination of 1% each of copper and iron sulfate
d) EDDS foam d with 1% copper sulfate
e) EDDS foam e with 1% iron sulfate
f) MGDA foam f with 1% copper sulfate
g) MGDA foam g with 1% iron sulfate
h) EDDS/MGDA foam with a combination of 1% each of copper and iron sulfate wherein the ratio of EDDS to MGDA is:

| | | | |
|---|---|---|---|
| i) | Combi 1 (4:1) | ii) | Combi 2 (2:3) |
| iii) | Combi 3 (3:2) | iv) | Combi 4 (4:1) |
| v) | Combi 5 (4.5:0.5 | vi) | Combi 6 (0.5:4:5) |

### Beaker Foam Results

The beaker foam results are tabulated on Tables 1, 2 and 3.

Table 1 shows that the beaker of EDTA alone (foam a) and EDDS alone (foam d) have low foam results demonstrating that these chelators complexed copper so as to suppress the copper catalyzed redox of hydrogen peroxide. The beaker of MGDA (foam f) has high foam results demonstrating that this chelate did not complex copper so that the copper catalyzed the redox of hydrogen peroxide.

**Table 1**

| | 1% EDTA | 1% EDDS | 1% MGDA |
|---|---|---|---|
| Swelling (copper) | 1x times (increase ~ 50ml) | 1x times (increase ~ 50ml) | 4x times (increase ~ 200ml) |

Table 2 shows that the beakers of EDTA alone (foam b) and MGDA (foam g) have low foam results demonstrating that these chelators complexed iron so as to suppress the iron catalyzed redox of hydrogen peroxide. The beaker of EDDS (foam e) has high foam results demonstrating that this chelate did not complex iron so that the iron catalyzed the redox of hydrogen peroxide.

**Table 2**

| | 2% MGDA | 1% EDDS | 1% EDTA |
|---|---|---|---|
| Swelling (iron) | 1x times (increase ~ 50ml) | 5x times (increase ~ 250ml) | 1x times (increase ~ 50ml) |

Table 3 provides the beakers of EDTA alone and various ratios of EDDS/MGDA (foams h(i)-h(vi). The EDDS/MGDA foams all produced low foam results demonstrating that these ratios of the chelator mixture complexed both of copper and iron so as to suppress the copper/iron catalyzed redox of hydrogen peroxide. The beaker of EDTA (foam c) has high foam results demonstrating that this chelate did not complex the copper/iron mixture because the iron competitively binds with EDTA so as to enable the copper catalyzed the redox of hydrogen peroxide.

**TABLE 3**

| Chelate Combination | EDTA | 1 | 2 | 3 | 4 | 5 | 6 |
|---|---|---|---|---|---|---|---|
| EDDS | X | 1.00% | 2.00% | 3.00% | 4.00% | 4.50% | 0.50% |
| MGDA | X | 4.00% | 3.00% | 2.00% | 1.00% | 0.50% | 4.50% |
| Swelling with copper & iron | 6x times (increase ~ 300ml) | 1x times (increase ~ 50ml) | 1x times (increase ~ 50ml) | 1x times (increase ~ 50ml) | 1x times (increase ~ 50ml) | 1x times (increase ~ 50ml) | 1x times (increase ~ 50ml) |

The study for aqueous copper (II) complexation in bleach shows that:
a) EDTA at 1% and EDDS at 1 wt% each produced a low foam height indicating effective copper (II) complexation;
b) MGDA at 1 wt% produced a high foam height indicating ineffective copper (II) complexation.

The study for aqueous iron (III) complexation in bleach shows that:
c) EDTA at 1 wt% and MGDA 2 wt% each produced a low foam height indicating effective iron (III) complexation;
d) EDDS at 1 wt% produced a high foam height indicating ineffective iron (III) complexation.

The study for complexation of a mixture of copper (II) and iron (III) in bleach shows that:
e) EDTA at 1 wt% produced a high foam height indicating ineffective complexation of the iron (III) and copper (II) mixture;
f) mixtures of EDDS/MGDA at variable wt% ratios of EDDS and MGDA produced low foam heights indicating effective complexation of the iron (III) and copper(II).

### Lift And Damage Study Protocol

The lift study determines the degree of lightness delivered to a colored hair swatch by bleaching with one or more experimental bleach compositions (based on the bleach formulation described above). The degree of lightness is determined by spectrophotometric analysis using a CIE Lab program. The analysis provides the hue and shade of the treated hair swatch on a CIE Lab scale in which the L* value indicates the lightness of the treated hair swatch with 100 being white or colorless and 0 being black. The lift or degree of lightness provided by the bleaching process is determined by comparing the L* value of the treated hair swatch with the L* value of the untreated hair swatch.

The damage study determines the degree of di-cysteine disulfide bond breakage of the keratin fibers caused by di-cysteine disulfide oxidation to cysteic acid. The degree of damage is determined by FTIR examination using an FTIR spectroscopic machine set to measure the peak absorption height at about 1042 cm⁻¹.

Brown hair swatches were purchased from a European laboratory cosmetics supply company which commercially supplies laboratory products for use in oxidative hair dye examinations. The brown hair swatches had been sanitized, shampooed and rinsed by the supplier and are used as is in the lift performance tests. While it is unknown whether residual salts such as calcium, copper and/or iron salts were present because of the rinse water used by the supplier, the lift results of the EDTA samples are comparable to historical EDTA lift results conducted on brown hair swatches that had been processed to remove any residual calcium, copper and/or iron salts. This historical comparison indicates that the supplied brown hair swatches were essentially free of such salts.

The protocol for bleaching the brown hair swatch involved applying the pro-bleaching formulation (bleach formulation with Welloxon) combined with selected chelate (chelate-bleaching formulation) to the brown hair swatch by placing it in a horizontal position on a glass plate and brushing the swatch with the semi-fluid bleaching composition described below to saturate the swatch. The glass plate with swatch was placed into a pre-heated oven at 40°C for 45 minutes to cause oxidation of the brown color of the swatch. The swatch on the glass plate was then removed from the oven, rinsed thoroughly with deionized water but was not treated with conditioner or shampoo as these treatment have the possibility of affecting the FTIR readings of the swatch. Following rinsing and drying the swatch was ready for color and FTIR analysis.

To analyze for color and damage, the treated hair swatch was placed on a spectrophotometric analysis plate, the plate with swatch placed into a spectrophotometer such as a Konica Minolta Illuminance Spectrophotometer CL-500 and the visible color spectrum analyzed according to a CIE Lab lightness, red, green, blue and yellow color mixture. The treated swatch was also placed into the Perkin Elmer Spectrum Two FTIR spectroscopic machine to determine disulfide bond scission by FTIR analysis of the extent of cysteic acid present in the treated sample. The FTIR spectrometer measured the absorption height of the sample at about 1042 reciprocal cm. For an explanation of the FTIR protocol, see the PDF publication by Thermo Fisher Scientific at https://assets.thermofischer.com/TFS-Asset/MSD/Application-Notes/forensic-analysis-ftir-microscopy-an56360-en.pdf.

The chelator-bleaching formulation used to treat the brown swatch was prepared by combining a sample of the above described bleach formulation with the Welloxon developer. The ratio of the bleach formulation to Welloxon developer in wt parts was 1:1.5 and the combination formed a pro-bleaching formulation. To the pro-bleaching formulation was added one chelator, EDTA, EDDS or MGDA or the two chelator combination of EDDS and MGDA to form the chelator-bleaching formulation. The chelator-bleaching formulation was composed of one of the chelator mixtures at one or more of the concentrations and ratios given on Tables 4, 5 and 6 above including:

| | | | |
|---|---|---|---|
| a) | EDTA | a) | EDDS |
| b) | MGDA | d) | EDDS/MGDA |

The Lift and Damage results should enable assessment of the influence of the chelator or mixture of chelators on the ability of the chelator-bleaching formulation to lighten the color from brown toward white and to damage the keratin fibers of the hair by forming additional cysteic acid.

Following the foregoing procedures for measuring the lift performance of bleaching formulations described above, chelator-bleaching formulations with chelators a) b), c) and d) were assessed at varying concentrations of the chelator or chelator mixture. These lift results are tabulated in Tables 4, 5 and 6. The first entry of each of Tables 4, 5 and 6 presents the lift produced by EDTA alone. The remaining entries of each table present the lift produced by the named chelator at the concentration given. When the named chelator lift entries are lower than the lift delivered by EDTA alone, they demonstrate that the concentration of the named chelator adversely affects the peroxide oxidation relative to the lift effect produced by EDTA. When named chelator lift entries are higher than the lift produced by EDTA alone, they demonstrate that the effect of the added chelator on peroxide oxidation is better than that caused by EDTA. The details of Tables 4, 5 and 6 are summarized as follows:
1) Lift results for the first entries of Tables 4, 5 and 6 are constant and show the lift caused by the chelator-bleaching formulation with EDTA.
2) For Table 4, the first entry shows the EDTA lift at 1 wt.% and the remaining entries show lift results for the chelator-bleaching formulation with EDDS. The lift results for the EDDS concentrations through 5 wt.% are much higher than the EDTA lift. Above 5 wt.% the EDDS lift drops to a level much below the EDTA lift.
3) For Table 5, the first entry shows the EDTA lift at 1 wt.% and the remaining entries show lift results for the chelator-bleaching formulation with MGDA. The lift results for the MGDA concentrations up to about 2 wt.% are lower than the EDTA lift. At about 2.5 wt% through 5 wt.% the lift results for MGDA are high relative to the EDTA lift.
4) For Table 6, the first entry shows the EDTA lift at 1 wt.% and the remaining entries of show lift results for the EDDS/MGDA combination at a constant total combination concentration of 5 wt%. The lift results for the EDDS/MGDA variable concentration range are substantially constant high lift relative to the EDTA lift. The optimum high lift occurs with the EDDS/MGDA ratio of 2:3 and 3:2.

The individual lifts produced by EDDS, Table 4 and MGDA, Table 5 can be combined to indicate what the combination, EDDS+MGDA produce for lift. Use of the total concentration and ratio range parameters for Table 6 (total of 5 wt.% and ratio beginning with lowest EDDS concentration) should give the combination lift results. These Combo Lift results (sum of the individual lifts divided by 2) is shown on the Hypothetical Table 6H.

Hypothetical Table 6H shows that the Combo Lift starts high relative to the EDTA lift and constantly decreases to become lower than the EDTA lift. In contrast, the actual Combo Lift for EDDS and MGDA shown by Table 6 is relatively constant across the concentration variations of EDDS and MGDA. This comparison of Table 6 with Table 6H demonstrates that the actual Combo Lift of Table 6 is not predictable.

**TABLE 4 EDDS**

| | | | | | | | | |
|---|---|---|---|---|---|---|---|---|
| **EDDS** | 0% | 0.50% | 1.00% | 2.00% | 3.00% | 4.00% | 5.0% | 7.70% |
| L* | 66.24 | 68.79 | 70.09 | 70.80 | 70.91 | 71.24 | 71.04 | 59.92 |
| L* increase relative to EDTA of first entry | 0 | +2.55 | +3.85 | +4.56 | +4.67 | +5 | +4.8 | -6.32 |

**TABLE 5 MGDA**

| | | | | | | | | |
|---|---|---|---|---|---|---|---|---|
| **MGDA** | 0% | 0.50% | 1.00% | 2.00% | 3.00% | 4.00% | 5.0% | 7.70% |
| L* | 66.24 (EDTA) | 63.87 | 64.99 | 66.36 | 69.37 | 71.88 | 73.31 | 72.04 |
| L* increase relative to EDTA of first entry | | -2.37 | -1.25 | +0.12 | +3.13 | +5.64 | +7.07 | +5.8 |

**TABLE 6 EDDS & MGDA**

| | | | | | | | | | |
|---|---|---|---|---|---|---|---|---|---|
| **EDDS** | 0% | 0.50% | 1.00% | 2.00% | 3.00% | 4.00% | 4.50% | 0.50% | 7.20% |
| **MGDA** | 0% | 4.50% | 4.00% | 3.00% | 2.00% | 1.00% | 0.50% | 7.20% | 0.50% |
| L* | 66.24 (EDTA) | 69.78 | 71.05 | 72.21 | 71.63 | 70.32 | 71.36 | 72.88 | 61.96 |
| L* increase relative to EDTA of first entry | 0 | +3.54 | +4.81 | +5.97 | +5.39 | +4.08 | +5.12 | +6.64 | -4.28 |

**Hypothetical Table 6H**

| | | | | | | | | | |
|---|---|---|---|---|---|---|---|---|---|
| Additive wt.% | EDDS | 0.5 wt.% | 1 wt.% | 2 wt.% | 3 wt.% | 4 wt.% | 5 wt.% | 7.7 wt.% | EDTA |
| to give 5 wt.% | MGDA | 4 wt.% | 4 wt.% | 3 wt.% | 2 wt.% | 1 wt.% | 0.5 wt.% | 0.5 wt.% | 1wt% |
| Lift | EDDS | 68.79 | 70.09 | 70.80 | 70.91 | 71.24 | 71.04 | 59.92 | |
| Lift | MGDA | 71.88 | 71.88 | 69.37 | 66.36 | 64.99 | 63.87 | 63.87 | |
| Combo Lift (Sum/2) | | 70.34 | 70.99 | 70.08 | 68.64 | 68.12 | 67.46 | 61.91 | 66.24 |
| L* increase relative to EDTA first entry | EDTA at 66.24 | +4.1 | +4.75 | +3.84 | +2.4 | +1.88 | +1.22 | -4.33 | EDTA first entry |

Following the foregoing procedures for measuring the FTIR results for chelator-bleaching formulations with chelators a), b), c) and d), keratin damage can be assessed. The Tables 7, 8 and 9 show FTIR damage caused by the chelator bleaching formulations at the same concentrations given by Tables 4, 5 and 6. The damage is indicated by the absorption height of the FTIR absorption at about 1042 reciprocal cm. A higher number (absorption) at this wave length indicates the presence of more cysteic acid and hence higher damage. The presence of cysteic acid is the result of the peroxide cleavage of the disulfide bonds of di-cysteine.

**TABLE 7**

| | | | | | | | | |
|---|---|---|---|---|---|---|---|---|
| **EDDS** | 0% | 0.50% | 1.00% | 2.00% | 3.00% | 4.00% | 5.0% | 7.70% |
| FTIR cm⁻¹ | 111.07 (EDTA) | 118.02 | 124.71 | 128.73 | 122.91 | 119.68 | 115.40 | 83.92 |

**TABLE 8**

| | | | | | | | | |
|---|---|---|---|---|---|---|---|---|
| **MGDA** | 0% | 0.50% | 1.00% | 2.00% | 3.00% | 4.00% | 5.0% | 7.70% |
| FTIR | 111.07 (EDTA | 115.66 | 115.17 | 121.62 | 115.44 | 133.55 | 129.62 | 101.13 |

**TABLE 9**

| | | | | | | | | | |
|---|---|---|---|---|---|---|---|---|---|
| **EDDS** | 0% | 0.50% | 1.00% | 2.00% | 3.00% | 4.00% | 4.50% | 0.50% | 7.20% |
| **MGDA** | 0% | 4.50% | 4.00% | 3.00% | 2.00% | 1.00% | 0.50% | 7.20% | 0.50% |
| FTIR | 111.07 (EDTA) | 116.89 | 124.83 | 118.44 | 118.68 | 115.43 | 117.09 | 98.20 | 104.59 |

### DAMAGE TO LIFT RATIO COMPARISONS

### EDDS LIFT AND FTIR DATA, TABLES 4 AND 7

| **TABLE 4** | | | | | | | | |
|---|---|---|---|---|---|---|---|---|
| **EDDS** | 0%* | 0.50% | 1.00% | 2.00% | 3.00% | 4.00% | 5.0% | 7.70% |
| L* | 66.24 | 68.79 | 70.09 | 70.80 | 70.91 | 71.24 | 71.04 | 59.92 |
| L* increase relative to base line at 0% | 0 | +2.55 | +3.85 | +4.56 | +4.67 | +5 | +4.8 | -6.32 |

| **TABLE 7** | | | | | | | | |
|---|---|---|---|---|---|---|---|---|
| **EDDS** | 0%* | 0.50% | 1.00% | 2.00% | 3.00% | 4.00% | 5.0% | 7.70% |
| FTIR | 111.07 | 118.02 | 124.71 | 128.73 | 122.91 | 119.68 | 115.40 | 83.92 |
| FTIR increase relative to EDTA base line | baseline | +6.95 | +13.64 | +17.66 | +11.84 | +8.61 | +4.33 | -27.15 |

### EDDS D TO L RATIO FOR USING TABLES 4&7

| | | | | | | | | |
|---|---|---|---|---|---|---|---|---|
| Damage to Lift ratio EDDS | 0% * | 0.50% | 1.00% | 2.00% | 3.00% | 4.00% | 5.0% | 7.70% |
| 7 ÷ 4 | 1.68 | 1.72 | 1.78 | 1.82 | 1.73 | 1.68 | 1.62 | 1.40 |
| Damage to lift ratio relative to EDTA as zero base line | 0 | +0.04 | +0.1 | +0.14 | +0.05 | 0.0 | -0.06 | -0.28 |
| D to L ratio relative to EDTA 3 baseline | 3 | 3.04 | 3.10 | 3.14 | 3.05 | 3.0 | 2.94 | 2.82 |

### MGDA LIFT AND FTIR, TABLES 5 AND 8

| **TABLE 5** | | | | | | | | |
|---|---|---|---|---|---|---|---|---|
| **MGDA** | 0%* | 0.50% | 1.00% | 2.00% | 3.00% | 4.00% | 5.0% | 7.70% |
| L* | 66.24 | 63.87 | 64.99 | 66.36 | 69.37 | 71.88 | 73.31 | 72.04 |
| L* increase relative to base line at 0% | | -2.37 | -1.25 | +0.12 | +3.13 | +5.64 | +7.07 | +5.8 |

| **TABLE 8** | | | | | | | | |
|---|---|---|---|---|---|---|---|---|
| **MGDA** | 0%* | 0.50% | 1.00% | 2.00% | 3.00% | 4.00% | 5.0% | 7.70% |
| FTIR | 111.07 | 115.66 | 115.17 | 121.62 | 115.44 | 133.55 | 129.62 | 101.13 |
| FTIR increase relative to EDTA base line | baseline | +4.59 | +4.1 | +10.55 | +4.37 | +22.48 | +18.55 | 9.94 |

### MGDA D TO L RATIO USING TABLES 5&8

| | | | | | | | | |
|---|---|---|---|---|---|---|---|---|
| Damage to Lift ratio MGDA | 0% * | 0.50% | 1.00% | 2.00% | 3.00% | 4.00% | 5.0% | 7.70% |
| 8 ÷ 5 | 1.68 | 1.81 | 1.77 | 1.84 | 1.66 | 1.86 | 1.77 | 1.40 |
| Damage to lift ratio relative to EDTA as zero base line | 0 | +0.13 | +0.11 | +0.16 | -0.02 | +0.18 | +0.11 | -0.28 |
| D to L ratio relative to EDTA 3 baseline | 3 | 3.13 | 3.11 | 3.16 | 2.98 | 3.18 | 3.11 | 2.82 |

### EDDS & MGDA COMBINATION, TABLES 6 & 9

| **TABLE 6** | | | | | | | | | |
|---|---|---|---|---|---|---|---|---|---|
| **EDDS** | 0%* | 0.50% | 1.00% | 2.00% | 3.00% | 4.00% | 4.50% | 0.50% | 7.20% |
| **MGDA** | 0%* | 4.50% | 4.00% | 3.00% | 2.00% | 1.00% | 0.50% | 7.20% | 0.50% |
| L* | 66.24 | 69.78 | 71.05 | 72.21 | 71.63 | 70.32 | 71.36 | 72.88 | 61.96 |
| L* increase relative to base line at 0% | 0 | +3.54 | +4.81 | +5.97 | +5.39 | +4.08 | +5.12 | +6.64 | -4.28 |

| **TABLE 9** | | | | | | | | | |
|---|---|---|---|---|---|---|---|---|---|
| **EDDS** | 0%* | 0.50% | 1.00% | 2.00% | 3.00% | 4.00% | 4.50% | 0.50% | 7.20% |
| **MGDA** | 0%* | 4.50% | 4.00% | 3.00% | 2.00% | 1.00% | 0.50% | 7.20% | 0.50% |
| FTIR | 111.07 | 116.89 | 124.83 | 118.44 | 118.68 | 115.43 | 117.09 | 98.20 | 104.59 |
| FTIR increase relative to EDTA base line | baseline | +5.82 | +13.76 | +7.37 | +7.61 | +4.36 | +6.02 | -12.87 | -6.48 |

### EDDS & MGDA D TO L RATIO USING TABLES 6&9

| | | | | | | | | | |
|---|---|---|---|---|---|---|---|---|---|
| Damage to Lift ratio EDDS and MGDA | 0% | 0.50% 4.5% | 1.00% 4% | 2.00% 3% | 3.00% 2% | 4.00% 1% | 4.5% 0.5% | 0.5% 4.5% | 7.70% 0.5% |
| 9 ÷ 6 | | 1.68 | 1.76 | 1.64 | 1.65 | 1.64 | 1.64 | 1.35 | 1.49 |
| Damage to lift ratio relative to EDTA as zero base line | EDTA D/L =1.68 | 0.0 | +0.08 | -0.04 | -0.03 | -0.04 | -0.04 | -0.33 | -0.19 |
| D to L ratio relative to 3 as baseline (EDTA) | 3.0 | 3.0 | 3.08 | 2.96 | 2.97 | 2.96 | 2.96 | 2.67 | 2.81 |

### Discussion of the Damage to Lift Ratios of Tables 4/7, 5/8 and 6/9

Tables 7, 8 and 9 demonstrate that except for the highest concentrations of the added chelator(s), all concentrations of EDDS, MGDA and EDDS/MGDA produced more cysteic acid than EDTA at 1 % produced (entry 1 of each of Tables 7, 8 and 9). This essentially universal increase in keratin fiber damage shown by Tables 7, 8 and 9 can be compared with the lift results of Tables 4, 5 and 6 to provide damage to lift ratios. Examination of the damage to lift ratios shows how these chelators perform overall relative to the EDTA damage to lift ratio.

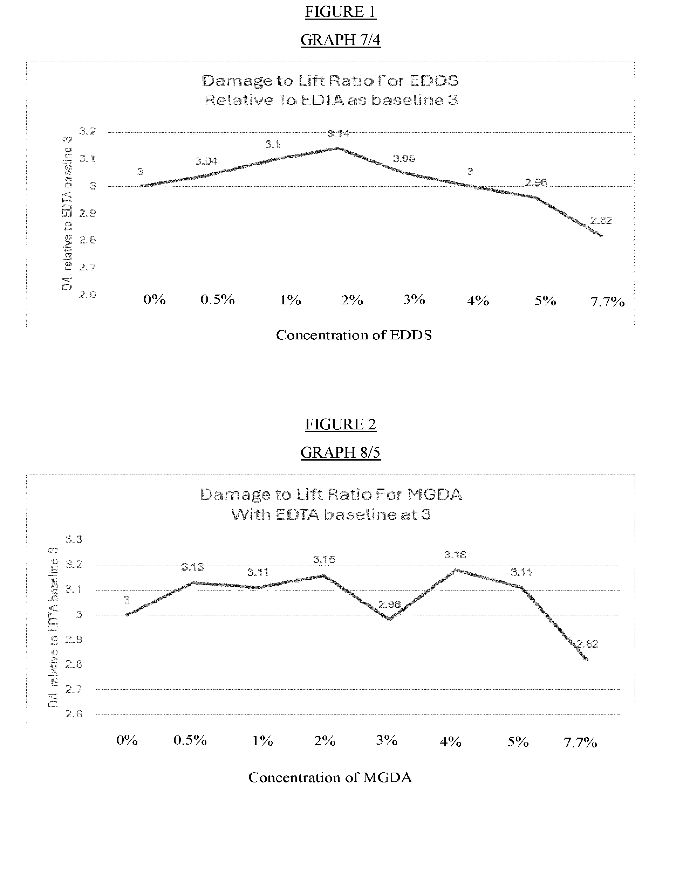

The damage to lift ratios are presented by Tables 4/7, 5/8 and 6/9. These ratios are presented graphically on Graphs 7/4, 8/5 and 9/6 of Figures 1, 2 and 3. The damage to lift ratio is determined by dividing the FTIR entry of Table 7, 8 or 9 by the corresponding Lift L* entry of Table 4, 5 or 6. The ratio range for each Table shows the variability of the damage versus lift ratio depending upon the chelator or chelator combination present and its or their concentration. For each Table, the first entry is the base line damage to lift ratio delivered by EDTA alone. The remaining entries for each Table show the relative damage to lift ratio for EDDS, MGDA and EDDS+MGDA.

With EDDS as the chelator for a fixed concentrations of oxidizer in the chelator-bleaching formulation on the hair swatch, the damage to lift ratio of Table 4/7, and depicted by Figure 1, Graph 7/4, shows an increase from 0 wt% EDDS to slightly less than 3 wt% and then shows a decrease to 7 wt%. This result shows that from zero to 3 wt% concentration of EDDS, the damage to lift ratio is negatively affected by EDDS even though Table 4 shows that EDDS increases the lift relative to the EDTA at these concentrations. Somehow, the peroxide keratin attack at this EDDS concentration range is significantly and affirmatively affected so that more keratin protein is damaged than occurs with EDTA alone. However, at a concentration of EDDS above 3 wt%, the damage to lift ratio decreases compared with the continued increase in lift shown by Table 4. This indicates a reversal of the affirmative peroxide keratin attack at the lower concentrations of EDDS. These results are not predictable. The reverse at mid-concentration is confirmed experimentally but is not predictable.

With MGDA as the chelator for fixed concentrations of oxidizer, the damage to lift ratio shown by Table 5/8 and depicted by Figure 2, Graph 8/5, jumps up and down throughout the range of 0.5 wt% to 5 wt% indicating that MGDA affects peroxide attack on keratin protein at some concentrations more than at others. This damage to lift progression is the result of incomprehensible high and low keratin protein damage. These results are not predictable.

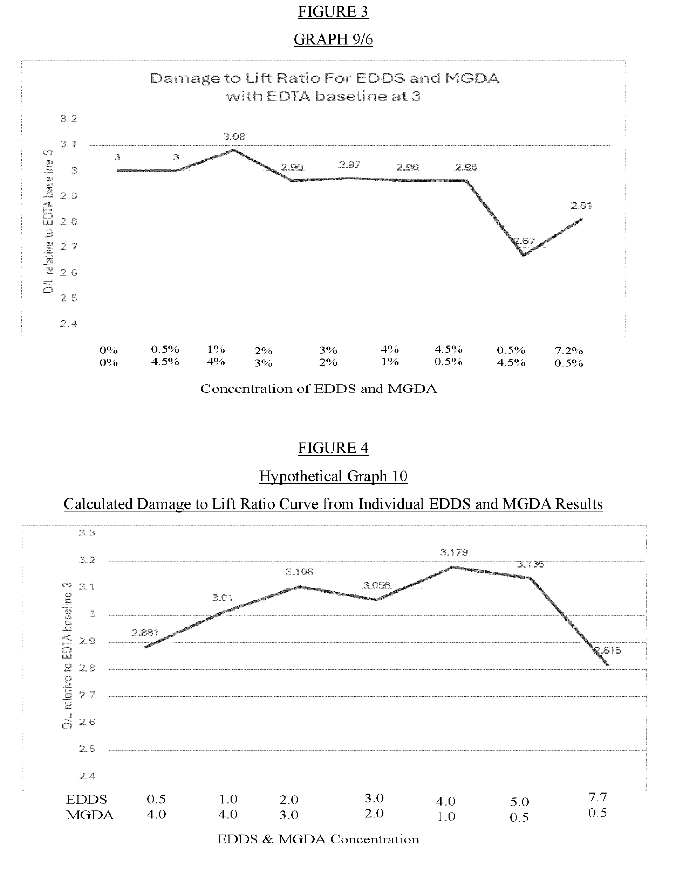

With the combination of EDDS/MGDA as the chelator mixture, the damage to lift ratio shown by Table 6/9 and depicted by Figure 3, Graph 9/6, is almost constant or flat throughout the ratio range of EDDS to MGDA. Given the highly variable damage to lift profiles exhibited by EDDS and MGDA alone, it might be expected that the combination would also exhibit a highly variable damage to lift profile. An analysis of the expectations from the combination of EDDS and MGDA can be garnered by combining the damage to lift ratio progressions for EDDS and MGDA as individual chelators.

The following hypothetical table and graph show this combination of the individual damage to lift progressions for EDDS and MGDA.

### Hypothetical Damage to Lift Ratio Progression for Combination of EDDS & MGDA Individual Progressions

The hypothetical damage to lift ratio progression for the combination of individual EDDS and MGDA progressions can be calculated by combining the lift and FTIR data for the individual results for EDDS and MGDA of Tables 4, 5, 7 and 8. The averaged combination of the lift results of Tables 4 and 5 yields the Hypothetical Lift progression for the EDDS, MGDA combination shown on Table 10 and depicted by Figure 4, Hypothetical Graph 10. Similarly, the averaged combination of the combining the FTIR results of Tables 7 and 8 yields the hypothetical damage progression for the EDDS, MGDA combination shown on Table 10.

The hypothetical comparison with the actual results of Table 6/9 combination and shown by Graph 9/6 can be made by beginning with the corresponding individual concentrations of EDDS and MGDA. Because Table 6/9 begins with EDDS at a 0.5 wt.% and MGDA at 4 wt.%, and traces through the increasing EDDS concentrations and decreasing concentrations of MGDA, this same concentration progression is taken from Tables 4, 5, 7 and 8. Hence, EDDS begins at 0.5 wt.% and ends at 7.7 wt.% while MGDA begins at 4.0 wt.% and ends at 0.5 wt.%. The sum of these two individual concentrations are as close as possible to the constant sum of 5 wt.% used for Table 6/9.

Hypothetical Table 10 presents these results. Hypothetical Graph 10, Figure 4, plots these D/L ratios of the EDDS-MGDA combination using the number 3 as the base line representing the D/L ratio delivered by EDTA alone.

### HYPOTHETICAL TABLE 10

| | | | | | | | | |
|---|---|---|---|---|---|---|---|---|
| Chelator wt.% to give 5 wt.% | EDDS | 0.5 wt.% | 1 wt.% | 2 wt.% | 3 wt.% | 4 wt.% | 5 wt.% | 7.7 wt.% |
| | MGDA | 4 wt.% | 4 wt.% | 3 wt.% | 2 wt.% | 1 wt.% | 0.5 wt.% | 0.5 wt.% |
| Lift | EDDS | 68.79 | 70.09 | 70.80 | 70.91 | 71.24 | 71.04 | 59.92 |
| Lift | MGDA | 71.88 | 71.88 | 69.37 | 66.36 | 64.99 | 63.87 | 63.87 |
| Sum/2 | | 70.34 | 70.99 | 70.08 | 68.64 | 68.12 | 67.46 | 61.91 |
| | | | | | | | | |
| Damage FTIR | EDDS | 118.02 | 124.71 | 128.73 | 122.91 | 119.68 | 115.40 | 83.92 |
| Damage FTIR | MGDA | 115.66 | 115.17 | 121.62 | 115.44 | 133.55 | 129.62 | 101.13 |
| Sum/2 | | 116.84 | 119.94 | 125.175 | 119.175 | 126.615 | 122.51 | 92.525 |
| | | | | | | | | |
| D/L | | 1.661 | 1.690 | 1.786 | 1.736 | 1.859 | 1.816 | 1.495 |
| Difference from EDTA D/L of 1.68 | | -0.019 | +0.01 | +0.106 | +0.056 | +0.179 | +0.136 | -0.185 |
| D/L ratio relative to 3 baseline | EDTA at 3.0 | 2.881 | 3.01 | 3.106 | 3.056 | 3.179 | 3.136 | 2.815 |

Comparison of Hypothetical Graph 10 and Graph 9/6 shows that the actual damage to lift results of Graph 9/6 (EDDS & MGDA combination) are not predictable. Hypothetical Graph 10 shows an increasing, ascending slope of the Damage to Lift ratio throughout the concentration variation. Graph 10 shows the expectation that the combination of EDDS and MGDA will exhibit an ever-increasing damage to lift progression. In other words, the damage result is predicted to become more severe relative to lift throughout this concentration variation. Table 6/9 shows, in contrast, that the progression of the Damage to Lift ratio of the EDDS/MGDA combination following the same two concentration gradients is essentially a flat line indicating an essentially constant Damage to Lift ratio throughout the concentration variation. These actual results of Graph 9/6 are not predictable from the Hypothetical Analysis.

Table 11 provides the CIE Lab and FTIR results for analysis of an EDTA bleaching formulation and an EDTA/EDDS/MGDA bleaching formulation. The tests are conducted with Cu present alone at 1%, Fe present alone at 1%, Ca present alone at 1%, and used as is from supplier (no prior rinse, no prior bleaching, no prior dyeing, no treatment with metal ions). The EDTA/EDDS/MGDA mixture was not at an optimum concentration ratio or at the optimum total concentration according to the Lift and the Damage to Lift results of Tables 4 - 6/9. The results show that the mixture of EDTA/EDDS/MGDA delivers a higher lift than EDTA alone for all metal ion tests and the "used-as-is" test. The EDTA/EDDS/MGDA mixture also delivers a lower damage to lift ratio than does EDTA against copper and against "used-as-is" even though the concentrations of EDTA, EDDS and MGDA are not optimized to effectively bind copper, iron and calcium. The higher amount of EDDS does not compensate for the lower amount of EDTA with respect to Ca because EDDS has a much lower stability constant for Ca than does EDTA. The low amount of MGDA does not compensate for the lower amount of EDTA with respect to iron because the MGDA is not at an optimum concentration of 2 wt.%. See footnotes 6, 7 and 8.

**Table 11**

| CIE Lab and FTIR Results | | | | | | | | | | |
|---|---|---|---|---|---|---|---|---|---|---|
| Product | EDTA bleaching formulation 1.0% EDTA | | | | | | EDTA/EDDS/MGDA bleaching formulation 0.5% EDTA, 1% EDDS, 0.5% MGDA⁵ | | | |
| pH (mixed) | ~10.4 | | | | | ~10.3 | | | | |
| n-tresses | 6 | | | | | 9 | | | | |
| L*a*b*3 | L* | a* | b* | FTIR² | D/L¹ | L* | a* | b* | FTIR² | D/L¹ |
| With Cu | 61.77 | 7.03 | 26.59 | 121.29 | 1.96 | 74.64 | 2.77 | 25.03 | 138.7 | 1.85⁶ |
| With Fe | 69.52 | 5.92 | 28.07 | 116.68 | 1.67 | 73.45 | 3.14 | 25.74 | 130.13 | 1.77⁷ |
| With Ca | 68.47 | 5.85 | 27.86 | 115.14 | 1.68 | 74.09 | 3.45 | 26.47 | 130.83 | 1.77⁸ |
| | | | | | | | | | | |
| Used as is from supplier⁴ | 65.27 | 7.06 | 29.68 | 115.41 | 1.77 | 71.82 | 4.54 | 27.73 | 119.00 | 1.66 |

| | | | | | | | | | | |
|---|---|---|---|---|---|---|---|---|---|---|
| 1) FTIR/ L*factor: the larger the factor the worse the damage to lift ratio. 2) FTIR is the absorbance of the cysteic acid signal at about 1042 cm⁻¹. 3) Using e-paint to provide a visual depiction of the L*ab value, the visual depictions of (a) and (b) are both brown, with (b) being a visibly lighter brown than the brown of (a). 4) No rinse, no Cu, Fe, Ca treatment. 5) Concentrations of all chelates not at optimum based on Table 6/9. MGDA optimum should be 2 wt.%. EDTA optimum should be 1 wt.%. 6) EDDS and EDTA have high stability constants for Cu. The concentration of EDDS binds more Cu so that no uncomplexed Cu is present to cause keratin fiber damage. 7) EDDS does not bind iron. MGDA and ETDA concentrations are too low to bind completely the iron concentration. Uncomplexed iron influences peroxide to cause higher keratin fiber damage. 8) EDDS has a low log stability constant for Ca while EDTA has a much higher log stability constant. The low concentration of EDTA and relatively higher concentration of EDDS means more uncomplexed Ca. Uncomplexed Ca influences peroxide to cause higher keratin fiber damage | | | | | | | | | | |

### Peroxide Oxidation Temperature Protocol

Using the brown hair swatch procedure and the above described experimental bleaching formulation with EDTA alone) and the chelator mixture of EDTA/EDDS/MGDA, the temperature increase produced by the redox reaction of the hair swatch can be determined. The bleaching formulation is prepared with 1 wt% copper sulfate and chelator EDTA alone, and chelator mixture of EDTA/EDDS/MDGA.

The brown hair swatch positioned on a horizontal glass plate is thoroughly saturated with the bleaching formulation with EDTA alone or the chelator mixture. A temperature probe with a wifi transmitter of the temperature is inserted into the hair swatch and the entire arrangement placed into a closed container at ambient temperature. The temperature increase broadcast by the wifi transmitter is recorded by a wifi receiver and recorder during a time period of about 1 hour. The temperature increase as a function of time is tabulated on Table 12 and shown on Graph 11.

**TABLE 12**

| | EDTA | Chelator mixture |
|---|---|---|
| Heat Rise °C | 62°C | 51°C |

### Temperature Rise Results

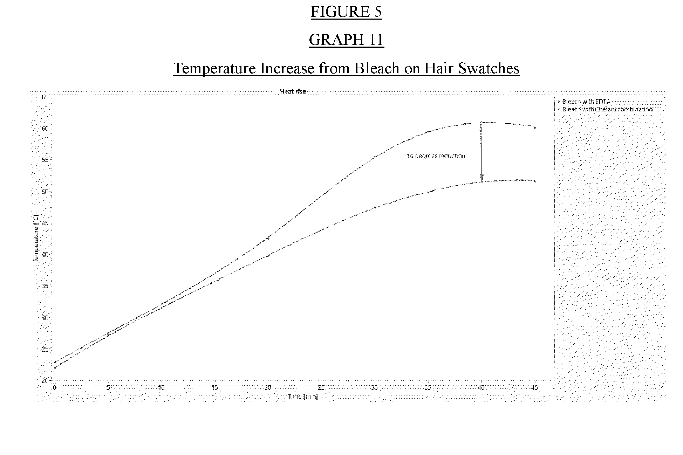

Table 12, Graph 11, Figure 5, shows temperature increase over time for bleaching formulation of EDTA alone and bleaching formulation mixture of EDTA/EDDS/MGDA. The temperature increase caused by bleaching formulation with EDTA alone achieves a maximum of 62°C, the temperature increase caused by bleaching formulation mixture of EDTA/EDDS/MGDA achieves a maximum of about 51°C. This difference in maximum temperature is significant for scalp and skin because the temperature of about 51 °C (124° F) delivers a "hot" feeling while a temperature of 62°C (144°F) blisters scalp and skin within seconds to minutes.

### CHELATE COMPLEXATION OF COPPER, IRON AND CALCIUM

To demonstrate the power of the three chelate mixture of EDTA, EDDS and MGDA, simple bench top runs can be conducted to show how the mixture of EDTA, EDDS and MGDA prevents dark coloration of a hydrogen peroxide solution of copper, iron and calcium. While many of these bench top runs are hypothetical, a few have been conducted to demonstrate the principles of this hypothetical example.

For a first run, a 50 ml solution of 6 wt.% hydrogen peroxide in water at a pH of about 9 may be prepared and is placed into a glass beaker. To this peroxide solution is added about 10 ml of 1 wt.% EDTA to form a peroxide-EDTA solution (PE solution). After about 5 minutes, about 10 ml of an aqueous solution of 0.5 wt.% copper sulfate is added to form a PEC solution. The PEC solution remains colorless. Then, about 10 ml of 0.5 wt.% aqueous calcium chloride is added to the PEC aqueous solution. The PEC solution will turn dark orange-red in about 10 to 20 seconds indicating formation of copper oxide resulting from decomposition of the hydrogen peroxide and formation of oxygen.

Another 50 ml portion of the PE solution is prepared. To this PE solution is added about 10 ml of 0.5 wt.% ferrous chloride. This solution contains only ferrous ions. The solution with ferrous chloride will remain colorless.

Another 50 ml portion of PEC (copper) solution is prepared. To this PEC solution is added about 10 ml of 0.5 wt.% ferrous chloride. This solution contains copper and iron ions. In about 5 to 10 seconds, the PEC solution with iron and copper will turn orange-red indicating formation of copper oxide.

Another 50 ml portion of PEC (Copper) solution is prepared. To this PEC solution is added about 10 ml of 0.5 wt.% ferrous chloride and 0.5 wt.% calcium chloride. This solution contains copper, iron and calcium ions. This PEC solution will turn dark orange-red to dark red indicating the formation of copper and ferric oxides.

For a second run, a 50 ml solution of 6 wt.% hydrogen peroxide in water at a pH of about 9 may be prepared and placed into a glass beaker. To this peroxide solution is added about 10 ml of 1 wt.% EDDS to form a peroxide-EDDS solution (PEDD solution). After about 5 minutes, 10 ml of an aqueous solution of 0.5 wt.% copper sulfate is added to form a PEDDC solution. The PEDDC solution will remain colorless. Then, about 10 ml of 0.5 wt.% aqueous calcium chloride is added to the PEDDC aqueous solution. This solution contains copper and calcium ions. The PEDDC solution will remain colorless.

Another 50 ml portion of the PEDD solution is prepared. To this PEDD solution is added about 10 ml of 0.5 wt.% ferrous chloride. This solution contains only iron ions. This PEDD with iron will turn dark red indicating formation of iron oxide.

Another 50 ml portion of the PEDD solution is prepared. To this PEDD solution is added 10 ml of 0.5 wt.% aqueous copper sulfate and calcium chloride. This solution contains copper and calcium ions and remains colorless. Then about 10 ml of 0,5 wt.% ferrous chloride is added. This solution with copper, calcium and iron will turn dark red indicating formation of iron oxide.

For a third run, a 50 ml solution of 6 wt.% hydrogen peroxide in water at a pH of about 9 may be prepared and placed into a glass beaker. To this peroxide solution is added about 10 ml of 1 wt.% MGDA to form a peroxide-MGDA solution (PMG solution). After about 5 minutes, about 10 ml of an aqueous solution of 0.5 wt.% copper sulfate is added. The solution will turn dark orange indicating formation of copper oxide.

Another 50 ml portion of the PMG solution is prepared. To this PMG solution is added about 10 ml of 0.5 wt.% ferrous chloride. This solution contains only iron ions. This solution with iron will remain colorless.

Another 50 ml portion of the PMG solution is prepared. To this PMG solution is added about 10 ml of 0.5 wt.% ferrous chloride. The solution will remain colorless. Then 10 ml of 0.5 wt.% aqueous copper sulfate is added. This solution contains iron and calcium ions and will turn dark orange indicating formation of copper oxide.

Another 50 ml portion of the PMG solution is prepared. To this PMG solution is added about 10 ml of 0.5 wt.% ferrous chloride and 0.5 wt.% calcium chloride. The solution will remain colorless. Then 10 ml of 0.5 wt.% of aqueous copper sulfate is added. The solution contains iron, calcium and copper ions and will turn dark orange to dark red indicating formation of copper oxide.

For a fourth run, a 50 ml solution of 6 wt.% hydrogen peroxide in water at pH of about 9 may be prepared and placed into a glass beaker. To this peroxide solution is added about 10 ml of 1 wt.% each of EDTA, EDDS and MGDA. Then addition of each of about 10 ml of 0.5 wt.% copper sulfate, about 10 ml of 0.5 wt.% ferrous chloride and about 0 ml of 0.5 wt.% calcium chloride is sequentially made. After each addition of the copper, ferrous and calcium portions, the solution will remain colorless indicating that the copper, iron and calcium ions will have been complexed so as to not interact with hydrogen peroxide to undergo oxidation.

While these hypothetical bench top runs have not been properly conducted to explore the parameters of full metal ion binding, a few of the runs have been conducted to demonstrate some of the results described.

### Analysis of Experimental Complexation Activity, Temperature Control and Advantages

The experiments described above demonstrate combinative complexation ability of the bleach composition of the invention against copper, iron and calcium ions. No single chelate successfully binds copper, iron or a mixture of copper and iron. Although EDTA individually binds iron and copper, EDTA fails when presented with a mixture of iron and copper. EDDS and MGDA individually are unable to bind both iron and copper yet their combination at practically any weight ratio binds the mixture of iron and copper. With EDTA, the combinative complexation ability against copper, iron and calcium ions increases the protection of keratin fibers against untoward oxidation catalyzed by copper and/or iron, lowers the maximum oxidation temperature, and increases the lightening or bleaching performance relative to standard EDTA performance.

Lift and damage are two signal indicators of performance of a bleaching composition. Combining the parameters of these indicators provides an understanding of the performance of the bleach, for better or worse. The combination of the tabular data of Tables 4-9 of the experimental section provides a ratio of damage to lift and depicts how damage compares with lift as a result of variations of the kind and concentration of chelate present relative to the baseline lift and damage delivered by use of EDTA alone. Graphs 7/4, 8/5 and 9/6 plot these damage to lift ratios of the chelates relative to the standard EDTA baseline.

Graphs 7/4 and 8/5 show the extreme variability of the damage to lift ratio progression for individual chelator bleaching formulations with EDDS and MGDA. EDDS alone exhibits a damage to lift ratio progression that shows severe damage as the concentration increases but inexplicably reverses at mid-concentration. MGDA alone exhibits a highly variable damage to lift progression that shows inexplainable high and low damage to lift ratios as concentration increases. When these two chelates are combined, the irrationality of their individual behavior is inexplicably smoothed as shown by Graph 9/6 to provide an essentially flat line damage to lift ratio throughout the variation of the ratio of EDDS to MGDA. The combination shown by Graph 9/6 is surprising in view of Graphs 7/4 and 8/5.

Moreover, the results of Graph 9/6 are not predictable as shown by the Hypothetical Damage to Lift progression determined by the combination of the individual Lift and Damage results for EDDS and MGDA. This Hypothetical Damage to Lift progression is discussed and tabulated in Table 10 of the Experimental section. Hypothetical Graph 10 plotting the data of Table 10 from the experimental section is attached Figure 4 . Comparison of Hypothetical Graph 10 and Graph 9/6 shows that the essentially flat line progression of the Damage to Lift ratio for the actual EDDS/ MGDA combination is not predictable from the ascending slope progression of combination of the lift and damage results for EDDS and MGDA individually.

The higher lift ability of the bleaching formulation with the combination of EDDS, MGDA and EDTA not only effectively binds copper, iron and calcium but also enables effectively improved lift relative to EDTA alone in the presence of these ions. Table 11 of the experimental section shows the improved effectiveness of this combination.

The experimental section above further demonstrates as shown by Table 12 that EDTA alone as the chelator in a bleach/copper ion composition tested on hair swatches causes the oxidation temperature to rise to a maximum slightly above 60°C while the mixture of EDTA/EDDS/MGDA causes the hair swatch oxidation temperature to rise to a maximum slightly below 50°C. This temperature difference compares to the difference between hot water and scalding hot water, e.g., 122° F versus 147 °F. This relation to skin reaction relative to these absolute temperatures show that this temperature control is highly significant with respect to protection of scalp and skin from scalding.

These discoveries are surprising especially in light of literature reports about individual chelation by EDTA, EDDS and MGDA as well as the expectations generated by the above described experiments using each of these chelants alone. Although it is not intended to be an explanation of the surprising effects produced by the EDTA/EDDS/MGDA combination, it is believed that the combination exhibits multiple chelator interactions not available to use of the individual chelators.

Bleaching of hair is conducted to remove the melanin color components such as eumelanin, phenomelanin and/or artificial dye products internal to the hair strands. The measure of the effectiveness of the bleach process is commonly referred to as lightening or lift. The degree of lightening or lift produced by a bleach is measured spectrophotometrically using a color scale such as the CIELAB color Space (Lab*) wherein L* represents the degree of lightness.

The ability of bleach to lighten hair color can be affected by the presence of metal ions on the hair. Copper, iron and calcium ions typically present on and in hair competitively complex with chelators: a) to cause loss of chelating effectiveness, b) to cause hydrogen peroxide decomposition leading to loss of lift, c) to cause excessive oxidation of keratin proteins, d) to increase the oxidation rate and consequent temperature of oxidation leading to hair damage and e) to discolor hair during the bleaching process. Salts of these metal ions are ubiquitous in potable water used for shampooing, rinsing and washing so that hair will always contain these metal ions and bleaching will be affected by the presence of these metal ions on the hair.

US Publication 2004/0086475 (hereinafter Pub '475) discusses the common knowledge that metal ions present on hair (from shampooing, showering and the like) are among the issues causing bleach damage to keratin fibers. Metal ions combine with hydrogen peroxide "to give highly damaging species such as free radicals which are believed to be very harmful to the disulphide bonds of hair" (¶0073), e.g., the keratin protein. Pub '475 explains that chelators complex with metal ions so that deleterious keratin fiber oxidation is prevented or minimized.

Pub '475 posits that although EDTA is best at preventing the copper catalyzed "highly damaging species of hydrogen peroxide", the presence of calcium ion interferes with this EDTA complexation of copper (¶0071). Pub '475 prefers EDDS (¶0080) because its copper to calcium constant ratio is much better than that of EDTA (¶0062).

The realistic picture, however, is not this simple.

The foaming experiments described above demonstrate that iron also interferes with copper complexation. While EDTA effectively binds copper and iron individually and EDDS and MGDA can bind one or the other of copper and iron, none of these chelators alone can bind a mixture of copper and iron. This result suggests that somehow iron is able to affect the complexation constants of these chelators. Surprisingly, we have found that a mixture of EDDS/MGDA/EDTA effectively complexes the copper/iron mixture.

Based on these foaming experiments and the known information about EDTA complexation constants toward copper and calcium, it might be expected that increasing the concentration of EDTA alone would provide effective complexation of a mixture of copper, iron and calcium while EDDS and MGDA individually would not be effective against this mixture. However, the lift and damage/lift experiments with EDTA, EDDS, MGDA below, show that their concentrations cannot be increased without limit to deliver lift. Beyond about 6% for any of these chelates or their mixtures, the lift ability decreases and the damage to lift ratio increases because of increased damage.

Faced with this seeming insoluble problem, we surprisingly discovered as shown by Table 11 that a combination of EDDS, MGDA and EDTA at the weight percent ratio range given above not only effectively binds copper, iron and calcium but also enables effectively improved lift relative to EDTA alone in the presence of these ions. We discovered that the combination of EDDS, MGDA and EDTA affects the interaction of these metal ions with peroxide and the color quotient of keratin fibers to improve the lift. Because EDTA binds each of copper, iron and calcium individually, it is expected that the combination would not generate a lift any greater than EDTA. In fact at a lower concentration of EDTA in the EDTA/EDDS/MGDA combination relative to EDTA alone, the complex improves lift in the presence of each of these metal ions as shown by Table 11. Moreover, the combination damage to lift ratio is lower against copper and lower for untreated hair relative to EDTA alone. While the combination damage to lift ratio is slightly higher against iron and calcium relative to EDTA alone, correction of the EDTA and MGDA concentrations of the non-optimal formulation of Table 11 will lower these damage to lift ratio.

This discovery is surprising in view of the fact that at the concentrations and ratios recited by the claims, the chelate mixture of EDTA, EDDS and MGDA should produce the same lift as EDTA alone when tested against these metal ions individually. Somehow, inexplicably, the inclusion of the three part chelator combination affirmatively influences the lift of peroxide oxidation of the color bodies of the brown hair swatches in the presence of any of these three metal ions. It is believed that this triple activity cannot be achieved with any of these chelants alone or with a combination of any two.

Finally, we discovered that while EDTA causes an unacceptable increase in hydrogen peroxide oxidation temperature which is deleterious to keratin fibers and harmful to skin and scalp, combination of the EDTA/EDDS/MGDA at the weight percent ratio range given above decreases this oxidation temperature to a point no longer deleterious to keratin fibers and skin and scalp. This result is both unexpected and surprising.

### STATEMENT OF EMBODIMENTS

The Statements of the Invention set forth further Embodiments of the Invention and provide details described above as well as additional details according to the Invention. The Statements supplement and expand the description of embodiments of the oxidative hair coloring composition and the corresponding method set forth in the Detailed Description. These Statements provide further disclosure of these aspects, features and parameters and may serve as claims of the invention. Discrepancies between the Statements and Detailed Description are to be considered to be additive rather than subtractive.

### Statements of Embodiments

1. A bleach composition comprising:
   a) an oxidizing agent;
   b) a chelate mixture consisting of ethylenediamine-N,N'disuccinic acid (EDDS), methylglycine-N,N-diacetic acid (MGDA) and ethylene diamine tetraacetic acid (EDTA wherein the weight parts ranges for EDDS, MGDA and EDTA in the chelate mixture relative to the total weight of the chelate mixture are:
      iv) EDDS at a weight parts range of about 4 to about 1; and
      v) MGDA at a weight parts range of about 3 to about 0.5; and
      vi) EDTA at a weight parts range of about 2 to about 0.5; and
   c) an alkalizing agent.
2. The bleach composition according to statement 1 wherein the chelate mixture ratio in weight parts for EDDS to MGDA to EDTA is any one of 4:0.5:0.5, 4:1:0.5, 4:2:0.5, 4:3:0.5, 3:0.5:0.5, 3:1:0.5, 3:2:0.5, 3:3:0.5,2:0.5:0.5. 2:1:0.5, 2:2:0.5, 2:3:0.5, or 1:0.5:0.5, 1:1:0.5, 1:2:0.5, 1:3:0.5; or 4:0.5:1, 4:1:1, 4:2:1, 4:3:1, 3:0.5:1, 3:1:1, 3:2:1, 3:3:1, 2:0.5:1, 2:1:1, 2:2:1 2:3:1, or 1:0.5:1, 1:1:1, 1:2:1, 1:3:1; or 4:0.5:1.5, 4:1:1.5, 4:2:1.5, 4:3:1.5, 3:0.5:1.5, 3:1:1.5, 3:2:1.5, 3:3:1.5, 2:0.5:1.5, 2:1:1.5, 2:2:1.5, 2:3:1.5, or 1:0.5:1.5, 1:1:1.5, 1:2:1.5, 1:3:1.5; or 4:0.5:2, 4:1:2, 4:2:2, 4:3:2, 3:0.5:2, 3:1:2, 3:2:2, 3:3:2, 2:0.5:2, 2:1:2, 2:2:2, 2:3:2, or 1:0.5:2, 1:1:2, 1:2:2, 1:3:2 or any weight parts fraction between these weight part ratios.
3. The bleach composition according to any of the preceding statements wherein the weight parts range for EDDS is a fraction between about 4 and about 1 including 4.0, 3.9, 3.8, 3.7, 3.6, 3.5, 3.4, 3.3, 3.2, 3.1, 3.0, 2.9. 2.8. 2.7, 2.6, 2.5, 2.4, 2.3, 2.2, 2.1, 2.0, 1.9, 1.8, 1.7, 1.6, 1.5, 1.4, 1.3, 1.2, 1.1, 1.0 or any fractional variation thereof.
4. The bleach composition according to any of the preceding statements wherein the weight parts range for MGDA is a fraction between about 3 and about 0.5 including 3.0, 2.9, 2.8, 2.7, 2.6, 2.5, 2.4, 2.3, 2.2, 2.1, 2.0, 1.9. 1.8. 1.7, 1.6, 1.5, 1.4, 1.3, 1.2, 1.1, 1.0, 0.9, 0.8, 0.7, 0.6, 0.5 or any fractional variation thereof.
5. The bleach composition according to any of the preceding statements wherein the weight parts range for EDTA is a fraction between about 0.5 and about 2.0 including 0.5, 0.6, 0.7, 0.6, 0.8. 0.9, 1.0, 1.1, 1.2. 1.3. 1.4, 1.5, 1.6, 1.7, 1.8, 1.9, 2.0 or any fractional variation thereof.
6. The bleach composition according to any of the preceding statements wherein the oxidizing agent comprises at least one peroxo compound at a wt.% of from about 20 wt.% to about 90 wt.%, preferably about 40 wt.% to about 90 wt.%, more preferably about 60 to about 90 wt.%.
7. The bleach composition according to any of the preceding statements wherein the oxidizing agent comprises at least one inorganic peroxo compound and/or organic peroxo compound.
8. The bleach composition according to any of the preceding statements wherein the oxidizing agent comprises a mixture of an inorganic peroxo compound and an organic peroxo compound with the inorganic peroxo compound being about 60 wt.% to about 98 wt.% of the total weight of the oxidizing agent of the bleach composition.
9. The bleach composition according to any of the preceding statements wherein the oxidizing agent comprises an inorganic peroxo compound alone, comprising one or more of a persulfate salt, a percarbonate salt, a perborate salt, hypochlorite salt, chlorite salt, hypobromite salt, bromite salt or any combination thereof and the cation of the salt is sodium, potassium or ammonium or any combination thereof.
10. The bleach composition according to any of the preceding statements wherein the organic peroxo compound comprises one or more of benzoyl peroxide, peroxybenzoic acid and halo derivatives thereof, pyridinium chlorochromate, peracetic acid, trifluoroacetic peracid, phthaloyl peroxide, t-butyl peroxybenzoate, t-butyl hydroperoxide, dicumyl peroxide, methyl ethyl ketone peroxide, methyl isobutyl ketone peroxide, acetone peroxide, acetyl benzoyl peroxide, di-(1-naphthoyl) peroxide, diacetyl peroxide or any combination thereof.
11. The bleach composition according to any of the preceding statements wherein the alkalizing agent comprises one or more of an alkaline metal salt, magnesium salt and/or ammonium salt of hydroxide, carbonate, bicarbonate, silicate, aluminosilicate, metasilicate, phosphate or any combination thereof.
12. The bleach composition according to any of the preceding statements further comprising one or more additives comprising at least one or more of a hair conditioner, a detergent, a fatty substance, a thickener, a rheologic agent, a particulate filler, a silicone polymer, a natural or non-natural amino acid, a humectant, an emollient, or any combination thereof.
13. The bleach composition according to any of the preceding statements further comprising one or more additives comprising:
   a) a hair conditioner selected from one or more of a cationic, nonionic or zwitterionic surfactant and any combination thereof;
   b) a detergent selected from an anionic surfactant salt comprising a C10-C30 saturated linear alkyl carboxylate, sulfate, sulfonate, isethionate, sarcosinate or its acyl derivative, taurate or its acyl derivative, or any combination thereof wherein the cation of the salt is sodium or potassium or a combination thereof;
   c) a fatty substance selected from one or more of a saturated branched or unbranched C10-C40 hydrocarbon, a C10-C40 saturated fatty acid, a saturated C6-C40 fatty alcohol, a C2-C12 alkyl mono-, di- or tri-ol ester of the C10-C40 saturated fatty acid, and any combination thereof;
   d) a thickener as a synthetic organic polymer selected from poly(meth)acrylic acid, poly(meth)acrylate, polyester, polyamide, polyol, acrylates copolymer, epoxy tosylamide resin, block copolymers thereof, and any combination thereof,
   e) a rheologic agent as a polysaccharide organic polymer selected from guar gum, xanthan gum, cellulose, starch, hydrolyzed starch, alginate, locust bean gum, carob gum, agar, carrageenan, gelatine, derivatives thereof wherein the derivative comprises substituents of the hydroxyl groups of the polysaccharide including methyl, ethyl, hydroxyethyl, hydroxypropyl, carboxylmethyl, hydroxpropylmethyl, and any combination thereof;
   f) a silicone polymer selected from linear or branched polydimethylsiloxane, amodimethylsiloxane, cage, cyclic or net polydimethylsiloxane, and any combination thereof;
   g) a natural or non-natural amino acid selected from glycine, arginine, aspartic acid, asparagine, tyrosine, glutamine, glutamic acid, histidine, proline, serine, tryptophan, methionine, alanine, homoalanine, valine, leucine, isoleucine, 3-amino propionic acid, 3-amino butyric acid, 4-amino butyric acid, 2-amino pentanoic acid, 3-amino pentanoic acid, 4-amino pentanoic acid, 5-amino pentanoic acid, 2-amino hexanoic acid, 3-amino hexanoic acid, 4-amino hexanoic acid, 5-amino hexanoic acid , 6-amino hexanoic acid or any combination thereof;
   h) a particulate filler comprising a diatomaceous earth, a bentonite, hectorite, zeolite, silica, montmorillonite, kaolin, mica, smectite, phyllosilicate ritellate or any combination thereof
   i) and any combination of a) - h).
14. The bleach composition according to statement 13 wherein the additive comprises one or more of b) detergent, c) fatty substance comprising a fatty alcohol and/or a hydrocarbon, e) thickener/rheologic agent, h) particulate filler or any combination thereof.
15. The bleach composition according to any of the preceding statements wherein the bleach composition is a solid, has a particulate or powder form and the total weight percentage of the chelate mixture relative to the total weight of the solid bleach composition is from about 4 wt% to about 16 wt%, preferably about 8 wt.% to about 16 wt.%.
16. The bleach composition according to any of the preceding statements wherein the bleach composition is a solid with a particulate or powder form, the total weight of the alkalizing agent is from about 10 wt% to about 60 wt%, preferably about 10 wt.% to about 40 wt.% relative to the total weight of the solid bleach composition.
17. The bleach composition according to any of the preceding statements wherein the bleach composition is a solid with a particulate or powder form, the total weight of the oxidizing agent is from about 20 wt% to about 90 wt%, preferably about 40 wt.% to about 90 wt.%, more preferably about 60 wt.% to about 90 wt.% relative to the total weight of the solid bleach composition.
18. The bleach composition according to any of the preceding statements wherein the bleach composition is a solid with a particulate or powder form, includes one or more additives, and the total weight percentage of the additive is the difference between 100% and the weight percentages of the oxidizing agent, the alkalizing agent and the chelate mixture.
19. The bleach composition according to any of the preceding statements wherein the bleach composition as a solid with a particulate or powder form is combined with one or more of a fatty substance comprising one or more of a saturated branched or unbranched C10-C40 hydrocarbon, a C10-C40 saturated or unsaturated fatty acid, a saturated or unsaturated C6-C40 fatty alcohol or any combination thereof to form a paste.
20. The bleach composition of statement 19 wherein the paste has a viscosity of about 5K to 10K mPas or approximating the viscosity of ketchup to peanut butter.
21. A kit comprising the bleach composition of any of the preceding statements contained in a first storage unit.
22. The kit according to statement 21 further comprising a second storage unit comprising a developer composition of at least hydrogen peroxide.
23. The kit according to statement 21 or 22 further comprising one or more of a third storage unit containing a portion of the detergent and fourth storage unit containing a portion of the fatty substance, preferably the fatty alcohol.
24. An aqueous bleaching formulation wherein the bleach composition of the first storage unit of the kit of statement 21 is dissolved in water to provide an aqueous beaching formulation wherein the total weight percentage of the chelate mixture relative to the total weight of the aqueous bleaching formulation composition is from about 3 wt % to about 6 wt%.
25. An aqueous bleaching formulation wherein the bleach composition of the first storage unit of the kit of statement 21 is dissolved in water to provide an aqueous bleaching formulation wherein the total weight percentage of oxidizing agent provides an oxidizing equivalent of from about 3 wt% to about 12 wt% of hydrogen peroxide in the aqueous bleaching formulation.
26. An aqueous bleaching formulation wherein the bleach composition of the first storage unit of the kit of statement 21 is dissolved in water to provide an aqueous bleaching formulation of a basic pH of from 9 to 11.
27. The aqueous bleaching formulation comprising the combination of the aqueous bleaching formulations of statements 24, 25 and 26 wherein the total amount of water added provides a wt:wt ratio of bleach composition to water of up to about 1:1 or up to about 1:2.
28. A peroxide bleaching formulation wherein the bleach composition of the first storage unit of the kit of statement 21 is combined with the developer of the second storage unit of the kit of statement 22 to provide a wt:wt ratio range of about 1:1 to about 1:2 of bleach composition to developer.
29. A peroxide bleach composition of statement 28 combined with the aqueous bleaching formulations of statements 24, 25 and 26 to provide a wt:wt ratio of peroxide bleach composition to water in the range of 1:1 to 1:2.
30. A method for bleaching hair comprising applying the aqueous bleaching formulation of any of statements 24, 25, 26 and 27 to the hair.
31. A method for bleaching hair comprising applying the peroxide bleaching formulation of statement 28 or 29 to the hair.
32. The method for bleaching hair according to any of preceding method statements 30 and 31 wherein the aqueous bleaching formulation or peroxide bleaching formulation causes an oxidation reaction on the hair to bleach the hair and the temperature of the oxidation reaction rises no higher than about 50°C.
33. The method for bleaching hair according to any of the preceding method statements wherein the pH of the aqueous bleaching formulation or peroxide bleaching formulation is from about 9 to about 11, preferably from about 9 to about 10.
34. The method for bleaching hair according to any of the preceding method statements wherein the pH of the aqueous bleaching formulation or peroxide bleaching formulation is adjusted to a pH of from about 9 to about 11, preferably from about 9 to about 10 by addition of an exogeneous amount of the alkaline agent.
35. The method for bleaching hair according to any of the preceding method statements wherein the lift of color lightening measured by the L scale of the CIELab color spectrum is approximately 10% to 15% higher than the color lightening produced by a bleaching formulation containing EDTA.
36. The method for bleaching hair according to any of the preceding method statements wherein the damage to lift ratio is substantially the same throughout the range of ratios of EDDS to MGDA to EDTA.
37. The method for bleaching hair according to any of the preceding method statements wherein the aqueous bleaching formulation or peroxide bleaching formulation remains on the hair for at least 5 minutes, preferably at least 2 minutes, and thereafter is rinsed with ambient temperature water.
38. The method for bleaching hair according to any of the preceding method statements wherein the aqueous bleaching formulation or peroxide bleaching formulation remains on the hair for at least 5 minutes, preferably at least 2 minutes, the hair is thereafter shampooed with an aqueous anionic surfactant detergent composition and thereafter rinsed with ambient temperature water.
39. A method for oxidatively dyeing hair comprising applying the aqueous bleaching formulation or peroxide bleaching formulation of any of the preceding method statements to the hair, rinsing the hair to substantially remove the remnants of the aqueous bleaching formulation or peroxide bleaching formulation and then applying to the hair an aqueous oxidative dye composition of at least one precursor, at least one coupler and at least hydrogen peroxide wherein the hydrogen peroxide concentration is from about 3 wt% to about 12 wt % relative to the total weight of the aqueous dye mixture.
40. The method for oxidatively dyeing hair according to any of the preceding method statements for oxidatively dyeing wherein the aqueous oxidative dye composition further includes sodium or potassium carbonate/hydrogen carbonate.
41. The method for oxidatively dyeing hair according to any of the preceding method statements for oxidatively dyeing further comprising a chassis of one or more of a phosphate anionic surfactant, a nonionic polyethoxyalkyl ether surfactant, a saturated or unsaturated fatty alcohol, a saturated or unsaturated fatty ester, a saturated or unsaturated fatty acid, a viscosity control agent, a C16-C32 saturated or unsaturated linear or branched hydrocarbon, a cationic conditioner, a humectant, an emollient, an antioxidant, and any combination thereof.
42. The method for oxidatively dyeing hair according to any of the preceding method statements for oxidatively dyeing wherein the precursor is one or more of 2-methoxymethyl-p-phenylene diamine, p-aminophenol, hydroxyethyl-p-phenylenediamine sulphate, hydroxypropyl bis(n-hydroxyethyl-p-phenylenediamine), 2-chloro-p-phenylenediamine, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diamino dihydroxypyrazolo pyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and addition salts, solvates and/or solvates of salts thereof.
43. The method for oxidatively dyeing hair according to any of the preceding method statements for oxidatively dyeing wherein the coupler is one or more of hydroxyethyl-3,4-methylenedioxyaniline, 2,4-diaminophenoxyethanol (DPE),; m-aminophenol (MAP), 4-amino-2-hydroxy toluene (AHT), ethyl ortho-amino phenol (EOAP), , 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 2-amino-5-ethyl phenol, 6-hydroxybenzomorpholine, 6-amino-m-cresol, 6-amino-o-cresol, 2-amino-4-hydroxyethylamino-anisole, 1,3-bis-(2,4-diaminophenoxy)propane, 2,6-dihydroxyethylaminotoluene, 2,4-diamino-1,5-di(2-hydroxyethoxy)benzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindoline, phenyl methyl pyrazolone, hydroquinone, 4-formyl-1-methylquinolinium-p-toluenesulfonate, and the addition salts, the solvates and/or solvates of the salts thereof.
44. The method for oxidatively dyeing hair according to any of the preceding method statements for oxidative dying wherein the coupler excludes any of resorcinol, alkyl resorcinol, alkyl chloro resorcinol, chloro resorcinol or any mixture thereof.
45. The bleach composition according to any of the preceding statements wherein the EDDS weight part range preferably is about 4 to about 1.5; more preferably about 4 to about 2.
46. The bleach composition according to any of the preceding statements wherein the MGDA weight part range preferably is about 3 to about 0.75; more preferably about 3 to about 1.
47. The bleach composition according to any of the preceding statements wherein the EDTA weight part range preferably is about 2 to about 0.75, more preferably about 1.5 to about 0.5.
48. The bleach composition according to any of the preceding statements wherein the concentration of the chelate mixture is from about 4 wt% to about 16 wt%, preferably about 8 wt% to about 16 wt% relative to the total weight of the bleach composition.
49. The bleach composition according to any of the preceding statements wherein the concentration of the chelate mixture is calculated to deliver from about 2 wt% to about 10 wt%, preferably about 3 wt% to about 8 wt%, more preferably up to about 7 wt%, most preferably up to about 6 wt. % of the chelate mixture when the bleach composition is diluted with water and/or developer to form an oxidizing liquid, paste or gel.

### MISCELLANEOUS STATEMENTS

The invention has been described broadly and generically herein. Each of the narrower species and subgeneric groupings falling within the generic disclosure also form part of the invention. This includes the generic description of the invention with a proviso or negative limitation removing any matter from the genus, regardless of whether or not the excised material is specifically recited herein. The inventions, examples, results and statement of embodiments described, stated and claimed herein may have attributes and embodiments include, but not limited to, those set forth or described or referenced in this application.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed and as provided by the statements of embodiments. Thus, it will be understood that although the present invention has been specifically disclosed by various nonlimiting embodiments and/or preferred nonlimiting embodiments and optional features, any and all modifications and variations of the concepts herein disclosed that may be resorted to by those skilled in the art are considered to be within the scope of this invention as defined by the appended claims and the statements of embodiments.

All patents, publications, scientific articles, web sites and other documents and ministerial references or mentioned herein are indicative of the levels of skill of those skilled in the art to which the invention pertains, and each such referenced document and material is hereby incorporated by reference to the same extent as if it had been incorporated verbatim and set forth in its entirety herein. The right is reserved to physically incorporate into this specification any and all materials and information from any such patent, publication, scientific article, web site, electronically available information, textbook or other referenced material or document.

The written description of this patent application includes all claims, examples and statements of embodiments. All claims and statements of embodiments including all original claims are hereby incorporated by reference in their entirety into the written description portion of the specification and the right is reserved to physically incorporate into the written description or any other portion of the application any and all such claims and statements of embodiments. Thus, for example, under no circumstances may the patent be interpreted as allegedly not providing a written description for a claim on the assertion that the precise wording of the claim is not set forth *in haec verba* in written description portion of the patent.

While the invention has been described in conjunction with the detailed description thereof, the foregoing description is intended to illustrate and not limit the scope of the invention, which is defined by the scope of the appended claims and the statements of embodiments. Thus, from the foregoing, it will be appreciated that, although specific nonlimiting embodiments of the invention have been described herein for the purpose of illustration, various modifications may be made without deviating from the scope of the invention. Other aspects, advantages, and modifications are within the scope of the following claims and the present invention is not limited except as by the appended claims and the statements of embodiments.

The specific methods and compositions described herein are representative of preferred nonlimiting embodiments and are exemplary and not intended as limitations on the scope of the invention. Other objects, aspects, and embodiments will occur to those skilled in the art upon consideration of this specification and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential.

The methods and processes illustratively described herein suitably may be practiced in differing orders of steps, and that they are not necessarily restricted to the orders of steps indicated herein or in the claims.

## Claims

1. A bleach composition comprising:
a) an oxidizing agent comprising at least one peroxo compound;
b) a chelate mixture consisting of ethylenediamine-N,N'disuccinic acid (EDDS), methylglycine-N,N-diacetic acid (MGDA) and ethylene diamine tetraacetic acid (EDTA wherein the weight parts ranges for EDDS, MGDA and EDTA in the chelate mixture relative to the total weight of the chelate mixture are:
i) EDDS at a weight parts range of about 4 to about 1; and
ii) MGDA at a weight parts range of about 3 to about 0.5; and
iii) EDTA at a weight parts range of about 2 to about 0.5; and
c) an alkalizing agent.

2. The bleach composition according to claim 1 wherein the chelate mixture ratio in weight parts for EDDS to MGDA to EDTA is any one of 4:0.5:0.5, 4:1:0.5, 4:2:0.5, 4:3:0.5, 3:0.5:0.5, 3:1:0.5, 3:2:0.5, 3:3:0.5,2:0.5:0.5. 2:1:0.5, 2:2:0.5, 2:3:0.5, or 1:0.5:0.5, 1:1:0.5, 1:2:0.5, 1:3:0.5; or 4:0.5:1, 4:1:1, 4:2:1, 4:3:1, 3:0.5:1, 3:1:1, 3:2:1, 3:3:1, 2:0.5:1, 2:1:1, 2:2:1 2:3:1, or 1:0.5:1, 1:1:1, 1:2:1, 1:3:1; or 4:0.5:1.5, 4:1:1.5, 4:2:1.5, 4:3:1.5, 3:0.5:1.5, 3:1:1.5, 3:2:1.5, 3:3:1.5, 2:0.5:1.5, 2:1:1.5, 2:2:1.5, 2:3:1.5, or 1:0.5:1.5, 1:1:1.5, 1:2:1.5, 1:3:1.5; or 4:0.5:2, 4:1:2, 4:2:2, 4:3:2, 3:0.5:2, 3:1:2, 3:2:2, 3:3:2, 2:0.5:2, 2:1:2, 2:2:2, 2:3:2, or 1:0.5:2, 1:1:2, 1:2:2, 1:3:2 or any weight parts fraction between these weight part ratios.

3. The bleach composition according to any of the preceding claims wherein the at least one peroxo compound comprises an inorganic peroxo compound alone, comprising one or more of a persulfate salt, a percarbonate salt, a perborate salt, hypochlorite salt, chlorite salt, hypobromite salt, bromite salt or any combination thereof and the cation of the salt is sodium, potassium or ammonium or any combination thereof; preferably a persulfate salt and/or a percarbonate salt.

4. The bleach composition according to any of the preceding claims wherein the alkalizing agent comprises one or more of an alkaline metal salt, magnesium salt and/or ammonium salt of hydroxide, carbonate, bicarbonate, silicate, aluminosilicate, metasilicate, phosphate or any combination thereof; preferably a carbonate, a silicate, a metasilicate or any combination thereof.

5. The bleach composition according to any of the preceding claims further comprising one or more additives comprising at least one or more of a hair conditioner, a detergent, a fatty substance, a thickener, a rheologic agent, a particulate filler, a silicone polymer, a natural or non-natural amino acid, a humectant, an emollient, or any combination thereof; preferably a detergent, a fatty substance, a thickener, a rheologic agent, a particulate filler or any combination thereof..

6. The bleach composition according to any of the preceding claims further comprising one or more additives comprising:
a) a hair conditioner selected from one or more of a cationic, nonionic or zwitterionic surfactant and any combination thereof;
b) a detergent selected from an anionic surfactant salt comprising a C10-C30 saturated linear alkyl carboxylate, sulfate, sulfonate, isethionate, sarcosinate or its acyl derivative, taurate or its acyl derivative, or any combination thereof wherein the cation of the salt is sodium or potassium or a combination thereof;
c) a fatty substance selected from one or more of a saturated branched or unbranched C10-C40 hydrocarbon, a C10-C40 saturated fatty acid, a saturated C6-C40 fatty alcohol, a C2-C12 alkyl mono-, di- or tri-ol ester of the C10-C40 saturated fatty acid, and any combination thereof;
d) a thickener as a synthetic organic polymer selected from poly(meth)acrylic acid, poly(meth)acrylate, polyester, polyamide, polyol, acrylates copolymer, epoxy tosylamide resin, block copolymers thereof, and any combination thereof,
e) a rheologic agent as a polysaccharide organic polymer selected from guar gum, xanthan gum, cellulose, starch, hydrolyzed starch, alginate, locust bean gum, carob gum, agar, carrageenan, gelatine, derivatives thereof wherein the derivative comprises substituents of the hydroxyl groups of the polysaccharide including methyl, ethyl, hydroxyethyl, hydroxypropyl, carboxylmethyl, hydroxpropylmethyl, and any combination thereof;
f) a silicon polymer selected from linear or branched polydimethylsiloxane, amodimethylsiloxane, cage, cyclic or net polydimethylsiloxane, and any combination thereof;
g) a natural or non-natural amino acid selected from glycine, arginine, aspartic acid, asparagine, tyrosine, glutamine, glutamic acid, histidine, proline, serine, tryptophan, methionine, alanine, homoalanine, valine, leucine, isoleucine, 3-amino propionic acid, 3-amino butyric acid, 4-amino butyric acid, 2-amino pentanoic acid, 3-amino pentanoic acid, 4-amino pentanoic acid, 5-amino pentanoic acid, 2-amino hexanoic acid, 3-amino hexanoic acid, 4-amino hexanoic acid, 5-amino hexanoic acid, 6-amino hexanoic acid or any combination thereof;
h) a particulate filler comprising a diatomaceous earth, a bentonite, hectorite, zeolite, silica, montmorillonite, kaolin, mica, smectite, phyllosilicate ritellate or any combination thereof;
i) any combination of a) - h); and
j) preferably the additive comprises one or more of a detergent of the anionic surfactant comprising the alkyl sulfate and/or alkyl sulfonate, a fatty substance comprising the fatty alcohol and/or the hydrocarbon, a rheologic agent and a particulate filler comprising silica, montmorillonite and/or mica and any combination thereof.

7. The bleach composition according to any of the preceding claims comprising a solid as a particulate or powder form or a paste form, and wherein:
a) the total weight percentage of the chelate mixture relative to the total weight of the solid bleach composition is from about about 4 wt.% to about 16 wt. %, preferably from about 8 wt% to about 16 wt%;
b) the total weight of the alkalizing agent is from about 10 wt% to about 60 wt%, preferably from about 10 wt.% to about 40 wt.% relative to the total weight of the solid bleach composition;
c) wherein the total weight of the oxidizing agent is from about 20 wt% to about 90 wt%, preferably about 40 wt.% to about 90 wt.%, more preferably about 60 wt.% to about 90 wt.%
d) relative to the total weight of the solid bleach composition.

8. A kit comprising the bleach composition of any of the preceding claims contained in a first storage unit and an optional second storage unit comprising a developer composition of at least aqueous hydrogen peroxide.

9. An aqueous bleaching formulation wherein the bleach composition of any of the preceding claims is dissolved in water to provide an aqueous beaching formulation wherein:
a) the total weight percentage of the chelate mixture relative to the total weight of the aqueous bleaching formulation composition is from about 3 wt. % to about 7 wt.%, preferably to about 6 wt.%;
b) the total weight percentage of oxidizing agent provides an oxidizing equivalent of from about 3 wt% to about 12 wt% of hydrogen peroxide in the aqueous bleaching formulation;
c) the pH of the aqueous bleaching formulation is from about 9 to about 11; and,
d) the total amount of water added provides a wt:wt ratio of bleach composition to water of up to about 1:1 to about 1:2.

10. A peroxide bleaching formulation wherein the bleach composition of any of the preceding claims is combined with at least a developer of hydrogen peroxide to provide the peroxide bleaching formulation wherein:
a) the total weight percentage of the chelate mixture relative to the total weight of the peroxide bleaching formulation composition is from about 3 wt. % to about 7 wt.%, preferably to about 6 wt.%;
b) the total weight percentage of oxidizing agent provides an oxidizing equivalent of from about 3 wt% to about 12 wt% of hydrogen peroxide in the peroxide bleaching formulation;
c) the pH of the peroxide bleaching formulation is from about 9 to about 11; and,
d) the total amount of developer composition added provides a wt:wt ratio of bleach composition to developer composition of up to about 1:1 to about 1:2.

11. A method for bleaching hair comprising applying the aqueous bleaching formulation of claim 9 or the peroxide bleaching formulation of claim 10 to the hair.

12. The method for bleaching hair according to claim 11 wherein the aqueous bleaching formulation or peroxide bleaching formulation remains on the hair for at least 5 minutes, preferably at least 2 minutes, the hair is thereafter or is shampooed with an aqueous anionic surfactant detergent composition before and thereafter rinsed with ambient temperature water.

13. A method for oxidatively dyeing hair comprising bleaching the hair according to the method of claim 12 and then applying to the hair an aqueous oxidative dye composition comprising:
a) an oxidizing component comprising hydrogen peroxide at a concentration is from about 3 wt% to about 12 wt % relative to the total weight of the aqueous dye mixture;
b) an alkalizing component comprising sodium or potassium carbonate/hydrogen carbonate;
c) a chassis of one or more of a phosphate anionic surfactant, a nonionic polyethoxyalkyl ether surfactant, a saturated or unsaturated fatty alcohol, a saturated or unsaturated fatty ester, a saturated or unsaturated fatty acid, a viscosity control agent, a C16-C32 saturated or unsaturated linear or branched hydrocarbon, a cationic conditioner, a humectant, an emollient, an antioxidant, and any combination thereof;
d) a precursor comprising one or more of 2-methoxymethyl-p-phenylene diamine (MBB), p-aminophenol (PAP), hydroxyethyl-p-phenylenediamine sulphate, hydroxypropyl bis(n-hydroxyethyl-p-phenylenediamine), 2-chloro-p-phenylenediamine, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diamino dihydroxypyrazolo pyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine, and addition salts, solvates and/or solvates of salts thereof;
e) a coupler comprising one or more of hydroxyethyl-3,4-methylenedioxyaniline (Aminol), 2,4-diaminophenoxyethanol (DPE),; m-aminophenol (MAP), 4-amino-2-hydroxy toluene (AHT), ethyl ortho-amino phenol (EOAP), 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 2-amino-5-ethyl phenol, 6-hydroxybenzomorpholine, 6-amino-m-cresol, 6-amino-o-cresol, 2-amino-4-hydroxyethylaminoanisole, 1,3-bis-(2,4-diaminophenoxy)propane, 2,6-dihydroxyethylaminotoluene, 2,4-diamino-1,5-di(2-hydroxyethoxy)benzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindoline, phenyl methyl pyrazolone, hydroquinone, 4-formyl-1-methylquinolinium-p-toluenesulfonate; and the addition salts, solvates and/or solvates of salts thereof and,
f) the coupler excludes any of resorcinol, alkyl resorcinol, alkyl chloro resorcinol, chloro resorcinol or any mixture thereof.

14. The method according to claim 13 wherein the precursor is at least MBB and/or PAP and the coupler is at least one or more of Aminol, DPE, MAP, AHT or EOAP or any combination thereof.

15. The bleach composition according to any of the preceding bleach composition claims wherein the EDDS weight part range preferably is about 4 to about 1.5; more preferably about 4 to about 2; and/or the MGDA weight part range preferably is about 3 to about 0.75; more preferably about 3 to about 1; and/or the EDTA weight part range preferably is about 2 to about 0.75, more preferably about 1.5 to about 0.5.

16. The use of the bleach composition of any of claims 1-7, 15 to bleach hair.
